# EUROPEAN PATENT APPLICATION

(11) **EP 4 510 155 A1**
(43) Date of publication of application: **19.02.2025**
(21) Application number: 24194946.0
(22) Date of filing: 16.08.2024
(51) Int. Cl.: H01F 41/02, H01F 41/04, A61B 5/06, A61B 34/20, B33Y 80/00

(54) **MAGNETIC FIELD SENSOR**

(30) Priority: 17.08.2023 US 202363533258 P
(71) Applicant: BIOSENSE WEBSTER (ISRAEL) LTD., 2066717 Yokneam (IL)
(72) Inventor: HAYAT, Nathanel Yehuda David, 2066717 Yokneam (IL); BERKOWITZ, Shemer Shmaryau, 2066717 Yokneam (IL); BREEN, Barry Neal, 2066717 Yokneam (IL); BAR-TAL, Meir, 2066717 Yokneam (IL); ZOHAR, Daniel, 2066717 Yokneam (IL); COHEN, Avraham, 2066717 Yokneam (IL)
(74) Representative: Carpmaels & Ransford LLP

(57) **Abstract**

Magnetic field sensors and methods for 3D-printing of the same are disclosed. The magnetic field sensor includes a monolithic body with a plurality of coils 3D-printed therein by successive printing of layers along a printing direction; the coils include longitudinally oriented coil(s) whose magnetic axe(s) are parallel to the printing direction and transversely oriented coil(s) whose magnetic axe(s) are perpendicular to the printing direction. The transversely oriented coil(s) is each 3D-printed with a magnetic channel that curves between a pair of opposite flux collection facets being perpendicular to its magnetic axis, such that it includes a magnetic core section extending along the printing direction. Each of the longitudinally and transversely oriented coils is 3D-printed with respective arrangement of conductive windings including a plurality of turns 3D-printed in planes of the 3D-printed layers. The turns of transversely oriented coil surround the magnetic core section to the magnetic channel thereof.

## Description

### CROSS-REFERENCE TO RELATED APPLICATION

This application claims the benefit of U.S. Patent Provisional Application 63/533,258, filed August 17, 2023, the contents of which are incorporated by reference as if set forth in its entirety herein.

### TECHNOLOGICAL FIELD

The present invention is in the field of magnetic field sensors and specifically relates to techniques of fabricating magnetic field sensors suitable for magnetic-field-based position sensing, particularly for sensing the positions of medical devices.

### BACKGROUND

In various therapeutic and diagnostic procedures, a catheter/probe is inserted into a patient's body (e.g., chamber of the heart) and to be brought into contact with a body tissue there. Typically, in such procedures, it is necessary to determine the catheter's location within the body (i.e., the location at which a distal tip of the catheter engages the body tissue) as well as the pressure applied thereby to the tissue.

Catheters having integrated location and pressure sensors for sensing the location of the catheter and the pressure/force applied thereby at the contact region with the tissue, are generally known. Such catheters typically utilize inductive coils for determining the location of the catheter within the body and/or the pressure/force applied thereby to a body tissue it engages with.

Conventional techniques for such catheters often utilize wire-winded coils to which a ferrite core may be manually inserted after the winding. The coils fabricated in this way are then soldered to a cable that carries the coil signal to processing circuits, and fitted within the catheter's body.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1A** illustrates a monolithic magnetic field sensor **100** with a plurality of coils **120** including longitudinally oriented coils **120L** and transversely oriented coils **120T** 3D printed therein, according to an embodiment of the present invention;
**Figs. 1B and 1C****,** illustrate respectively a longitudinally oriented coil **120L** and a transversely oriented coil **120T,** which may be included/3D-printed in the monolithic magnetic field sensor **100** of **Fig. 1** according to some embodiments of the present invention;
**Figs. 1D and 1E** respectively illustrate the magnetic channels of the #D printed coils illustrated in **Figs. 1B and 1C****;**
**Figs. 2A, 2B, 2C and 2D** illustrate several views of a transversely oriented coil **120T** with magnetic channel curved/folded a helical-like shape, which may be 3D-printed/included in the monolithic magnetic-field/positioning sensor **100** according to some embodiments of the present invention;
**Figs. 3A, 3B and 3C** illustrate several views of a transversely oriented coil **120T** having magnetic channel **129T** with a multi-helix shape (double helix in this example), which may be 3D-printed/included in the monolithic magnetic-field/positioning sensor **100** according to some embodiments of the present invention;
**Fig. 4A** illustrates a transversely oriented coil **120T** with magnetic channel curved/folded a meander-like shape, which may be 3D-printed/included in the monolithic magnetic-field/positioning sensor **100** according to some embodiments of the present invention;
**Fig. 4B** illustrates a cross-sectional view of the coil of **Fig. 4A****;**
**Figs. 5A and 5B** illustrate several views of a longitudinally oriented coil **120L** with magnetic channel curved/folded in a coiled-like shape that wrapped about the conductive windings, which may be 3D-printed/included in the monolithic magnetic-field/positioning sensor **100** according to some embodiments of the present invention;
**Fig. 6A** depicts a side-view of a medical instrument **1000** furnished with a monolithic *Five Axes* magnetic-field based positioning sensor **100** according to an embodiment of the present invention;
**Fig. 6B** depicts a perspective view exemplifying the internal structure of the magnetic field sensor **100** furnished in the medical instrument **1000;**
**Fig. 6C** depicts a perspective view exemplifying the flux collectors' configuration of the magnetic field sensor **100** furnished in the medical instrument **1000** according to some embodiments of the invention;
**Fig. 6D** depicts a perspective view exemplifying an arrangement of electric contacts/terminals of the longitudinally and transversely oriented coils of the magnetic field sensor **100** arranged at an external surface thereof;
**Figs. 6E and 6F** illustrate respectively the back and front sides of a signal connector configured for connecting/coupling with the electric contacts/terminals of the magnetic field sensor **100** exemplified in **Fig. 3D;** and
**Fig. 7** is a flow diagram of a method **200** to fabricate a magnetic-field/positioning sensor 100 by 3D-printing technology according to some embodiments of the present invention.

### DETAILED DESCRIPTION OF EMBODIMENTS

There is a need in the art of catheters, for physically agile and sensitive catheters having compact/narrow dimensions, that are capable of providing accurate feedback on the location of the catheter in the body, and the contact interface between the catheter and the tissue and the force (e.g., vector) applied between them.

U.S. patent No. U.S.8,535,308 and U.S. patent application No. U.S. 2020/015693, which are both assigned to the assignee of the present Application, disclose for example configurations of such catheters utilizing sensory circuits having several coils, for sensing the location and orientation of the catheter body, as well as the force applied thereby at the interface with the tissue.

One challenge in the art of such catheters is to fit accurate location and force sensors within the narrow body of the catheter, whose lateral dimensions are typically only several millimeters. Indeed, as indicated above, in conventional catheters technologies, the location and force sensors generally utilize miniature wire-winded coils to which a ferrite core may be manually inserted with an open magnetic circuit configuration in order to yield sufficient induced-voltages suitable for location and force sensing of the catheter components. These conventional techniques are however time consuming and costly and involve delicate manual operations for fitting of the coils within the small dimensions of the catheter body.

Therefore, there is a need in the art for more efficient, cost-effective techniques for fabrication of such catheters and for the fabrication of small high sensitivity coils (i.e., having high induced voltage in response to magnetic fields) suitable for use for location and or force sensing in catheters such as those disclosed above. Moreover, there is a need in the art for automated fabrication techniques of such coils, suitable for mass production with yield of standardized coils having less variability between fabricated coils of the same design. Further, in the interest of further advent in the art of catheters, there is also a need in the art to facilitate even smaller and/or of higher impedance/induced-voltage, so as to improve either or both of the sensitivity and agility, and/or reduced dimensions, of the catheter.

The present invention provides a novel positioning sensory system and novel configurations and fabrication methods for magnetic field sensors including open magnetic circuit coil(s) suited for magnetic sensors of high induced-voltage, for example for use as position sensors to be furnished within narrow dimensions of a catheter body.

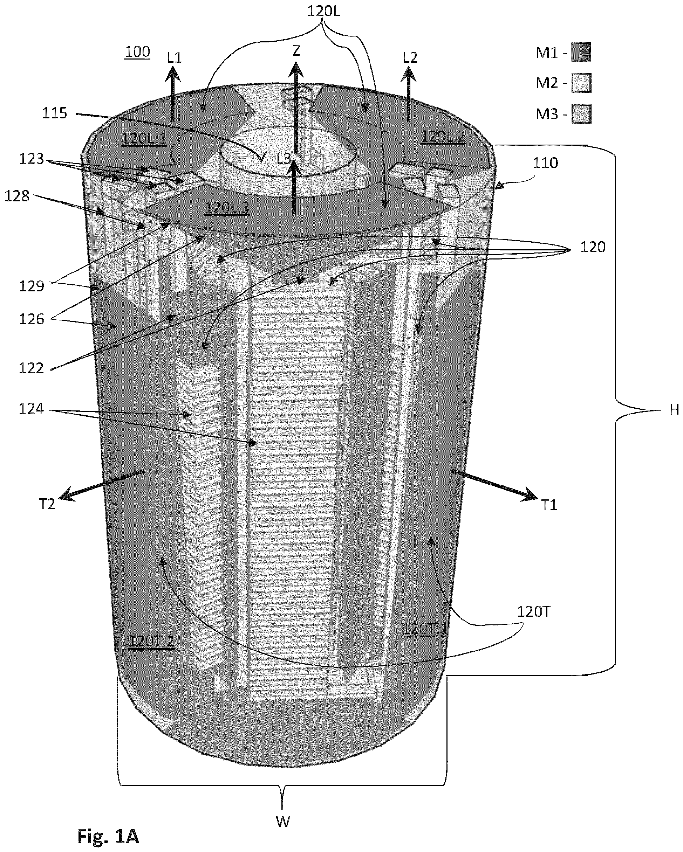

**Figs. 1A to 1E** illustrate a magnetic field sensor **100** (e.g. multi-axis magnetic-field based positioning sensor) according to an embodiment of the present invention. The magnetic field sensor **100** includes a monolithic body **110** with a plurality of coils **120** defined/fabricated therein by multi-material 3D printing of successive printed layers along a printing direction **Z.**

According to the technique of the invention the magnetic field sensor **100** is fabricated by 3D printing of at least three different 3D-printable materials **M1 to M3** including:
- At least one type of magnetic material **M1** which is used/3D-printed to form the magnetic channel(s) **129** of the coils **120,** which typically includes magnetic cores **122** of the coils **120** and typically also magnetic flux collector **126** thereof, as described below.
- At least one type of conductive (generally none-magnetic) material **M2** which is used/3D-printed to form a conductive channel **128** of the coils **120** including conductive windings **124** and contact terminals **123** thereof; and
- At least one type of non-magnetic, and typically dielectric/non-electrically-conductive material **M3** which is used/3D-printed to form a bulk of the monolithic body **110** of the sensor **100** at regions from which the magnetic and conductive materials are excluded.
Spacings between adjacent windings/turns **124** are occupied by 3D printed non-electrically-conductive material. The non-electrically-conductive material may for instance a non-magnetic dielectric material **M3** which is 3D printed in the bulk of the monolithic body **110** and/or the magnetic material **M1** used for printing the magnetic channels in cases where it is none-electrically conductive.

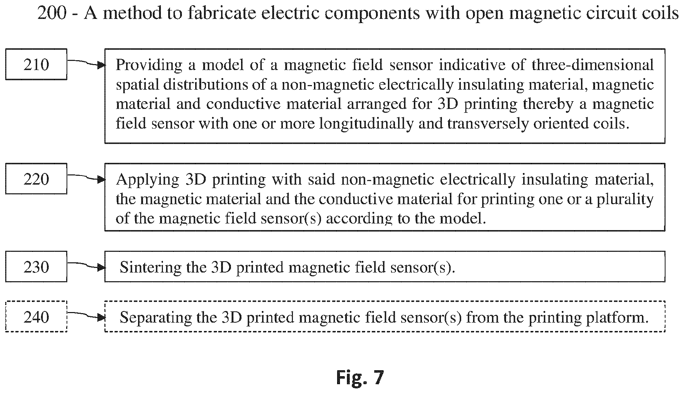

According to some aspects of the present invention, the sensor **100** is fabricated utilizing 3D printing method (additive manufacturing technology) such as that described in more details with reference to **Fig. 7** below. The 3D printing method includes a layer by layer deposition of at least three printable materials **M1 to M3** along a printing direction **Z.** Typically the at least 3 types of printable materials include: magnetic material(s) **M1** used for printing the magnetic channels **129** of the coils **120** (including the magnetic cores **122** and the flux collectors **126** thereof); conductive material(s) **M2** for printing the electric channels **128** of the coils (including the conductive windings **124** and the electric contacts **123);** and non-magnetic electrically insulating (dielectric) material, which is 3D printed to form the bulk of the monolithic body **110** of the sensor **100,** as well as typically/optionally to fill the spacings between the windings **124** of the coils to electrically insulate between them.

The terms *magnetic- material* and/or *particles* is used herein to designate materials of high relative magnetic permeability µᵣ, for example having relative permeability preferably of µᵣ ≥100 relative to the vacuum permeability µ₀, and more preferably in some cases in the order of µᵣ ≥500, or even in the order of µᵣ ≳5000. Conversely the terms *non-magnetic- material* and or particles are used herein to designate materials of low relative permeability µᵣ which is substantially lower than that of the magnetic materials used in the electronic components' fabrication and typically in the order of µᵣ ~ 1.

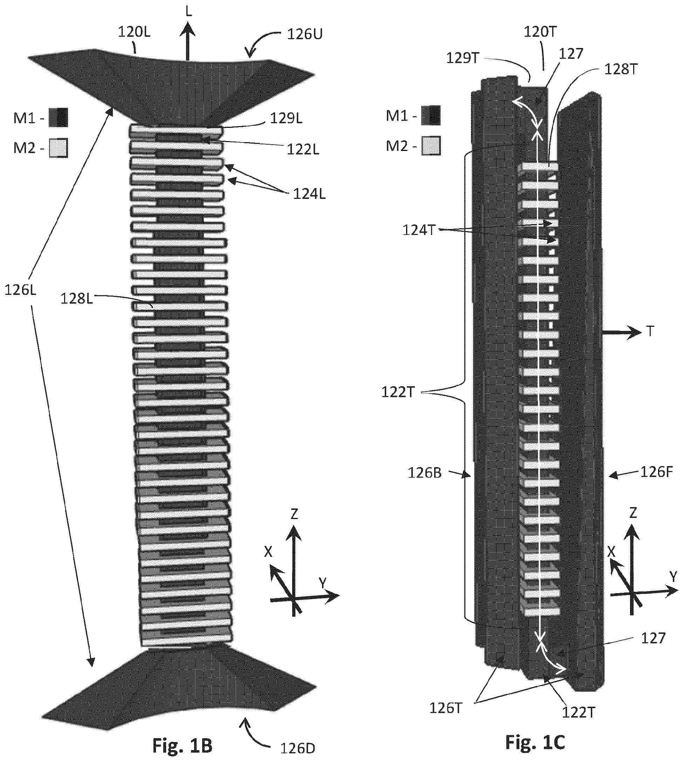

In **Figs. 1A to 6B****,** various 3D printed aspects/parts of the magnetic field sensor **100** are illustrated, with respective legend indicating the colors-/shades- codes that are used in each figure to designate the different 3D-printable materials **M1** to **M3** used in the printing of each aspect/part. In this regard it is noted that in some figures (e.g. in **Figs. 1B to 5B** **and** **6B****)**, the bulk material **M3** of the monolithic body **110,** is not specifically illustrated so as not to obscure the figures. Also, in some figures the same material, such as the magnetic material is marked with two different colors-/shades-codes in order to emphasize/distinct between different functional parts/sections made of the same material.

The plurality of coils **120** in the example of the magnetic field sensor **100** illustrated in **Figs. 1A to 1E** include one or more longitudinally oriented coils **120L** and one or more transversely oriented coils **120T.** The phrases *longitudinally oriented coils* and *transversely oriented* coils are used herein to designated coils whose respective magnetic axes are respectively oriented substantially parallel to the printing direction **Z** and transversely relative to the printing direction **Z** respectively.

Each of the coils **120** includes at least one electric channel **128,** 3D printed in the monolithic body **110** with conductive material **M2.** The electric channel **128** is 3D printed to include a pair of electric contacts 123 at opposite end of the electric channel **128** and an arrangement of conductive windings **124** electrically connected in between the pair of electric contacts **123** and including a plurality of conductive turns of the coil. The electric channel **128** is thus configured and operable to convert magnetic flux passing through the coil's windings **124** (along the coil's respective magnetic axis), to induced voltage at the electric contacts **123** thereof. The electric channel **128** is typically configured such that electric contacts **123** are arranged from one side of the monolithic body **110,** so that electrical connection thereto can be made efficiently, e.g. by one electrical connector, and/or to facilitate fitting of the electric components in narrow spaces without a need to access different sides thereof with electrical/signal connection lines.

Each of the transversely oriented coils **120T** and typically (optionally) also each of the longitudinally oriented coils **120L** also includes at least one magnetic channel **129** 3D printed in the monolithic body **110** with magnetic material **M1** which typically has relative permeability µᵣ in the order of hundred(s) or more.

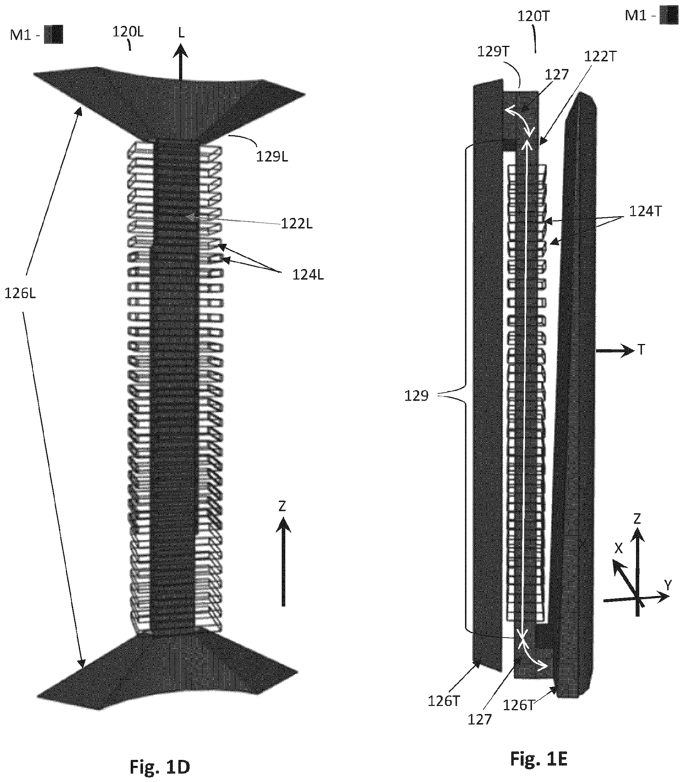

The magnetic channel **129** is 3D printed to include respective magnetic core **122** surrounded by the conductive windings **124** of the electric channel **128** of their respective coils. The magnetic channel **129** is thus configured and operable direct/channel, and optionally concentrate, external magnetic flux which is directed along the coil's respective magnetic axis, to flow/pass through the coil's windings **124,** to improve/increase the induced voltage at the electric contacts **123** of the coils and hence the sensitivities thereof. The magnetic channel **129** of each of the transversely oriented coils **120T** also includes magnetic flux collectors **126T** coupled from opposite ends of the magnetic channel **128** and having relatively large flux collection facets/surfaces **126F** and **126B** facing the magnetic axis (e.g. **T1** and/or **T2)** of their respective transversely oriented coil **120T.** In longitudinally oriented coils **120L** having a magnetic channel **129,** the magnetic channel **129** also typically magnetic flux collectors **126L** coupled from opposite ends of the magnetic core **122** and having relatively large flux collection facets/surfaces **126U** and **126D** facing the longitudinal magnetic axis (e.g. **L1, L2** and/or **L3)** of the respective coil. **Figs. 1D** and **1E** exemplify and illustrate the magnetic channels **129L** and **129T** of a longitudinally and transversely oriented coils respectively. For clarity the magnetic channels **129L** and **129T** are shown surrounded by outlines marking the conductive windings **124L** and **124T** of the longitudinally and transversely oriented coils respectively.

**Figs. 1B** and **1C** illustrate respectively, examples of a longitudinally oriented coil 120L and a transversely oriented coil **120L** fabricated in the monolithic body **110** of the sensor 100. Fig. 1B exemplifies a longitudinally oriented coil 120L which includes a magnetic channel 129L defining a magnetic core of the coil 122L formed/3D printed with magnetic material **M1**, and an electric channel 128L including conductive windings 124L having a plurality of conductive turns of surrounding the magnetic core 122L of the coil 120L. Fig. 1C exemplifies a transversely oriented coil 120T which includes a magnetic channel 129T defining a magnetic core of the coil 122T formed/3D printed with magnetic material M1 and an electric channel 128T including conductive windings 124T having a plurality of conductive turns surrounding the magnetic core 122T of the coil 120T.

It should be noted that the magnetic channel 129 of any one or more of the coils 120 not necessarily defines a single magnetic path (e.g. passing between the pair of flux collectors **126** of the coil) and in some embodiments it may be split in the form of plurality magnetic subchannels defining a plurality of magnetic cores for the respective coil, each being surrounded by the conductive windings **124** thereof. Similarly, it should be noted that the electric channel **128** of any one or more of the coils **120** not necessarily defines a single conductive path between the pair of electric contacts **123** of the coil and in some embodiments, it may be split in the form of plurality electric subchannels defining a plurality of winding sets surrounding the same or different sections of the magnetic core **122** of the coil.

In some embodiments the 3D printing technology used for the sensor's **100** fabrication provides for printing layers with thicknesses that are substantially smaller than the minimal feature sizes that can be printed within the layers (in other words: the interlayer resolution is substantially higher than the resolution of in-layer features/pixels that can be 3D printed; or the layer pitch is higher than the minimal pitch of in layer features/pixels). For instance, in certain non-limiting examples the sensor **100** may be printed with 3D printing technology providing transversal, in-layer, minimal feature sizes of about 24-50 micrometers sintered (µm) along each of the lateral X and Y axes, while with much smaller thicknesses of the printed layers of about 9-10 µm sintered. Note that here the minimal in-layer feature sizes were considered to be twice the lateral sizes of the pixels, whose pitches are about 12-25 µm). This is because practically, in some cases/embodiments, the functional in-layer features of the sensor (e.g., conductive turns and/or an electrically insulative material between them) are printed with lateral width of at least two pixels in order for the printing to be reliable and robust against misprints of in layer pixels. In such embodiments, planarly printing the conductive turns within the printed layers facilitates achieving about/more-than three times the turn count/density as compared to that achievable by printing the turns perpendicularly-to/across the printing layers.

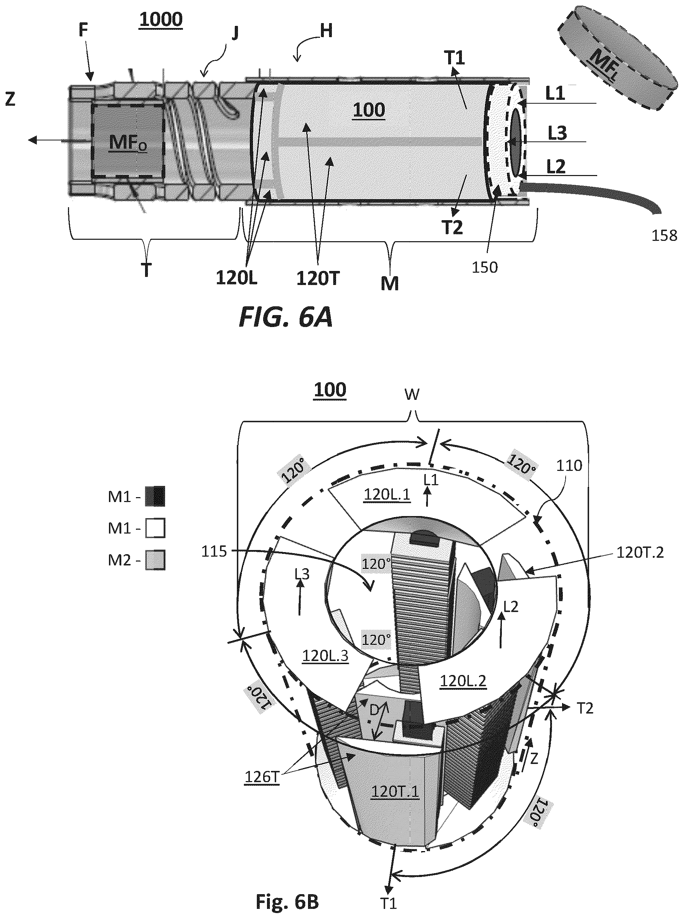

Moreover, in some embodiments the magnetic field sensor **100** is fabricated with an elongated shape of its monolithic body **110** such that its length **H** along the longitudinal-axis/printing-direction **Z** is substantially larger than its characteristic lateral width **W** (e.g. the aspect ratio H/W>>1) to facilitate the fitting of the sensor **100** within narrow a catheter as illustrated for instance in **Fig. 6A****.** For example, in some embodiments the characteristic lateral width **W** of the sensor **100** is about 2mm, and its length **H** is about 4 mm. In such embodiments, curving the magnetic channel **129** of the transversely oriented coil(s) **120T** to extend along the printing direction **Z** and printing a stack of conductive turns of the coils' windings **124T** stacked along the printing direction **Z** and, such that each being planarly 3D printed within the printed layers to surround the magnetic channel **129,** facilitates fabrication of a higher count of conductive turns and accordingly better sensitivity of the transversely oriented coil(s) **120T.**

Indeed, for the longitudinally oriented coils, such as **120L** illustrated in **Fig. 1B****,** whose magnetic axes **L** (e.g. in this example **L1, L2,** and **L3)** are parallel to the printing direction **Z** and which are designated to sense magnetic flux propagating along the printing direction **Z,** printing the conductive turns of the coils' windings **124L** planarly within the printed layers, may be relatively straight forward, since by when the turns of the conductive windings **124L** are in planar orientation in the printing layers, they inherently lay in planes that is substantially perpendicular to magnetic flux axes **L** of the longitudinally oriented coils **120L** (at the magnetic axes **L** which are parallel to the printing direction **Z).**

However, mere printing of the turns of the conductive windings **124T** of the transversely oriented coils **120T** with planar orientation in the printing layers is not sufficient to facilitate sensing of magnetic flux propagating along the magnetic axis **T** of a transversely oriented coils **120T,** since the magnetic axis **T** of a transversely oriented coil, such as **120T** illustrated in **Fig. 1C****,** is directed transversely (e.g. perpendicular) to the printing direction **Z,** and in order to capture this flux, the turns of the conductive windings **124T** should conventionally be arranged perpendicular to the magnetic axes **T.**

Therefore, to overcome this drawback and facilitate printing the turns of the conductive windings **124T** of a transversely oriented coil **120T** in planar orientation in said printing layers to yield higher count/density of turns, each transversely oriented coil **120T** is **3D** printed in the monolithic body **110** with a magnetic channel **122T** that includes at least a pair of opposite flux collectors **126T** defining/having respective, relatively large facets/surfaces, **126F** and **126B,** facing-the magnetic axis **T** of the transversely oriented coil **120T** (e.g. substantially perpendicular to the magnetic axis **T),** such that the magnetic channel **129T** curves/folds **127** such that one or more sections **122T** thereof, functioning as the magnetic core of the transversely oriented coil **120T** extends along a direction perpendicular-to/intersecting the magnetic axis **T** of the transversely oriented coil **120T.** Accordingly with magnetic channel **129T** being curved/folded **127** such that it magnetic core **122T** extends substantially perpendicularly to (e.g. in direction intersecting) its magnetic axis **T,** magnetic flux from the direction of the transverse magnetic axis **T** of the coil can be collected by flux collectors **126T** and channeled through the magnetic core **122T** that extends perpendicularly thereto. In turn the turns of the conductive windings **124T** of a transversely oriented coil **120T** (at least some of them), are printed in planar orientation within the printing layers such that they surround the magnetic core **122T** and can capture/sense the magnetic flux that is channeled to path therethrough from the direction of the transversely oriented magnetic axis **T** of the transversely oriented coil **120T.** In other words, the magnetic channel **129** is configured and operable for collecting magnetic fields flux of directed substantially along the magnetic axis **T** of the transversely oriented coil**120T,** via the facets **126F** and **126B** of the flux collectors **126T,** and channeling that flux to flow via the magnetic core **122T** which extends substantially perpendicularly (or more generally in an intersecting direction) relative to the magnetic axis **T.** In other words, the core geometry flips the magnetic field propagating internally in the magnetic core **122T,** by 90° relative to the external magnetic field.

In this manner, the technique of the present invention facilitates 3D printing the turns of the conductive windings **124** of both the longitudinally and transversely oriented coils, **120L** and **120T,** planarly within the planes of the printed layers (i.e., in planes substantially perpendicular to the printing direction **Z),** and thus yielding higher count of turns and improved sensitivity of the coils **120 /** sensor **100.**

In this regard it should be noted that according to some embodiments, the flux collector **126T** and in particular their facets/surfaces **126F** and **126B** that face the magnetic axis **T** of the transversely oriented coil **120T,** are typically made wider (e.g. with substantially larger area) than the characteristic width (e.g. cross section area) of the magnetic channel **122T** between them. Even more specifically the areas of the flux collectors facets/surfaces **126F** and **126B,** are typically made substantially larger than the characteristic cross section area of the magnetic core section(s) **122T** of the magnetic channel **129** which extend perpendicularly to the magnetic axis **T,** so that the flux collectors **126T** collect much more magnetic field flux from the direction of the magnetic axis **T** than the amount of flux that would be collected to the magnetic channel from other directions, thus making transversely oriented coil **120T** sensitive to magnetic field components directed along the magnetic axis **T,** while negligibly sensitive to magnetic field components parallel to **T.** Optionally the flux collectors **126T** and/or other sections of the magnetic channel are tapered towards the magnetic core sections **122T** (e.g. to facilitate efficient flux channeling from the relatively wide flux collection facets/surfaces **126F** and **126B** thereof to the narrower magnetic channel **122T).**

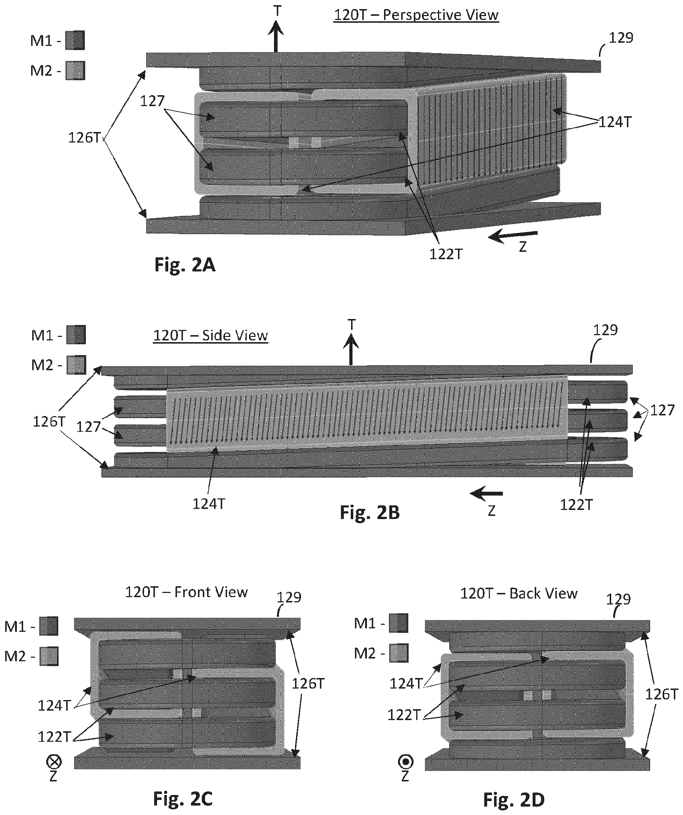

In **Figs. 1A to 1E** one optional configuration of the transversely oriented coils **120T** printed in the magnetic sensor **100** is exemplified. The magnetic channel **129T** of that transversely oriented coils **120T** being curved/folded **127** such that one section extends substantially along the printing direction to serve as the magnetic core **122T of** the coil and is being surrounded by conductive turns **124T** that are each planarly printed in the printing layers (e.g. the turns being substantially perpendicular to the printing direction). **Figs. 2A to 4B** provide depictions of several other non-limiting examples of configurations of the transversely oriented coils **120T** whose magnetic channels **122T** are curved/folded to have several sections **122T** thereof extending substantially along (e.g. substantially parallel-to) the printing direction and surrounded by conductive turns **124T** to serve as the magnetic core **122T** thereof. Like reference numerals are used in **Figs. 2A to 4B** to designate elements/sections of similar functionality as in the transversely oriented coils described above with reference to **Figs. 1A to 1E****.**

**Figs. 2A to 2D** respectively exemplify perspective- side-, front- and back- views of a transversely oriented coil **120T** which may be printed in the magnetic sensor **100** according to embodiments of the present invention. In this example the magnetic channel **129T** is curved/folded **127** several times such that between the flux collectors **126T,** the magnetic channel **129T** is printed-with/has a helical-like shape with a plurality of section thereof **122T** extending substantially along the printing direction Z and serving as magnetic cores of the coil. The plurality of section serving as the magnetic cores **122T** are surrounded by turns of the conductive windings **124T** of the conductive channel which are planarly printed in the printing layers with relatively small pitch/distances between them. The turns of the conductive windings **124T** of the conductive channel **128** are typically spaced from one another by electrical insulative material printed in the layer between them (e.g. the electrically insulative material between the turns may be the bulk material **M3** and/or optionally in some places it may be the magnetic material **M1** in case it is electrically insulative and will not magnetically short the flux propagation along the magnetic core). As shown the magnetic channel **129T** is formed in a helical like shape such that the multiple magnetic core sections **122T** thereof traverse through the conductive windings **124T** to channel magnetic flux that flows between the flux collectors several times through the conductive windings **124T** and thereby provide improved sensitivity of the coil. Although in this non-limiting example the magnetic channel **129T** is folded such that two magnetic core sections **122T** thereof traverses through the windings **124T,** it should be appreciated by those versed in the art that the coil **120T** may generally be printed with other configurations of the helical channel and may be formed with any suitable number of curves/folds **127** to yield any suitable number of magnetic core sections through the windings that provide the optimized sensitivity for the specific sensory application for which it is used.

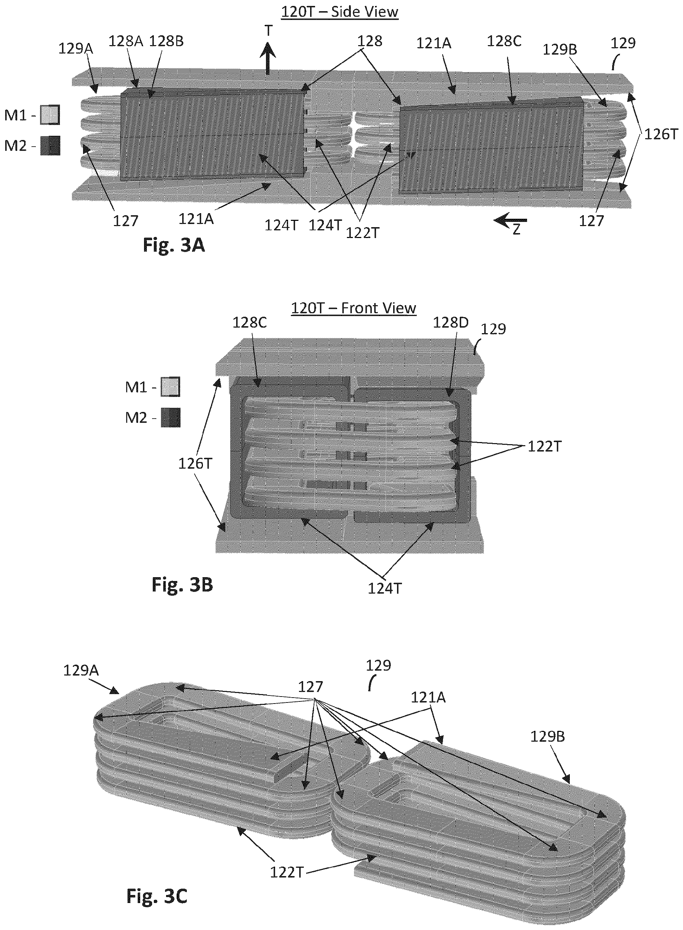

**Figs. 3A to 3C** respectively exemplify side- and front views, and specifically a portion of the magnetic channel **129** of a transversely oriented coil **120T,** which may be printed in the magnetic sensor **100** according to embodiments of the present invention. In this example between, the flux collectors **126T,** the magnetic channel **129T** is split into a plurality of magnetic subchannels (two in this example **129A** and **129B)** each being curved/folded **127** several times in the form of a helix (or otherwise a helical-like shape), such that the magnetic channel **129T** has a multi-helix shape (double helix in this example - one helix formed by each of the magnetic subchannel **129A** and **129B).** The magnetic are printed with the curves/folds **127** such that a plurality of sections **122T** thereof extend substantially along to the printing direction **Z** to serve as magnetic core sections **122T** of the coil **120T** and are surrounded by turns of the conductive windings **124T** of the coil **120T.** In this non-limiting example, the conductive windings **124T** include several winding sets **128A** to **128D** each including a plurality of conductive turns surrounding different one or more of the magnetic core sections **122T.** To this end, in various embodiments, the electric channel **128** between the electric contacts (shown as **123** in **Figs. 1A** to **1E****),** may be printed as a single conductive path passing between the pair of electric contacts **123** and shaped to form the winding sets **128A** to **128D** along a single conductive path, or the electric channel **128** between the electric contacts may be printed to split into several (e.g. 4) electric sub channels (e.g. which may be connected to one another in series; e.g. to maximize the induced voltage in the electric channel **128)** and each forming a plurality of windings/turns of the one or more winding sets **128A** to **128D.**

As indicated above with reference to **Figs. 1A to 1E****,** in certain embodiments of the present invention the magnetic channel **129** has a tapered shape/structure in a region thereof between the flux collection surfaces/facets of the flux collectors **126** and the magnetic core sections **122.** The example illustrated in **Figs. 3A to 3C** exemplifies such tapered shape of the magnetic channel **129** in which the cross-section area/width of the magnetic channel **129** is getting narrower along the direction advancing from the flux collection surfaces/facets of the flux collectors **126** towards the core sections **122** of the channel. In this non-limiting example, the tapering of the magnetic channel **129** is made gradually at several location including tapering of the flux collectors **126,** as shown in the tapered regions **121A** marked in **Fig. 3A****,** as well as tapering of the section **121B** of the magnetic channel **129** that directed the magnetic flux between the flux collectors to the core sections **122** as illustrated in **Fig. 3C****.** The tapering provides for improved coupling of external magnetic flux from the magnetic axis **T** of the coil that is collected by the flux collectors **126** to flow through the magnetic channel, and thereby improves the sensitivity of the coil. It should be noted that although in this non-limiting example, the magnetic channel tapering is exemplified for the transversely oriented coil **120T** and is illustrated in several tapered regions **121A** and **121B,** in general tapering of the magnetic channel towards the core sections may be implemented alternatively or additionally also for the longitudinally oriented coils **120L** of the sensor **100** illustrated in **Fig. 1A****.**

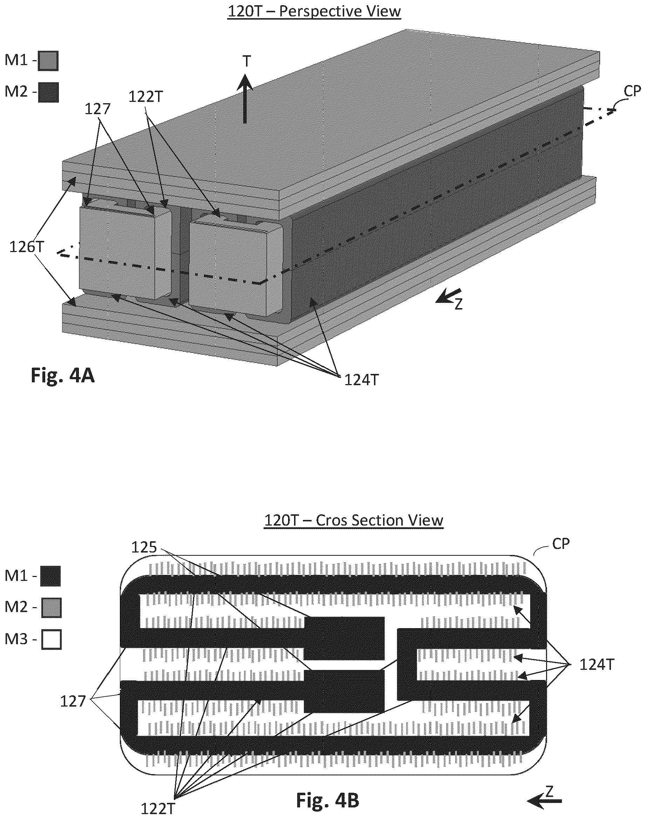

**Figs. 4A and 4B** exemplify a transversely oriented coil **120T,** which may be printed in the magnetic sensor **100** according to yet another embodiment of the present invention. **Fig. 4A** depicts a perspective view of a transversely oriented coil **120T** and **Fig. 4B** depicts a cross-sectional view of the coil taken along a plane **CP.** In this example between the magnetic channel **129** extends in a meander-like shape/pass between the flux collectors **126T.** The although flux collectors **126T** are not visible in **Fig. 4B****,** the regions **125** of the magnetic channel **129** to which the flux collectors **126T** are coupled is illustrated in the figure. The magnetic channel **129** is printed with the curves/folds **127** such that a plurality of sections **122T** thereof extend substantially along to the printing direction **Z** to serve as magnetic core sections **122T.** Accordingly the magnetic core sections **122T** are surrounded by turns of the conductive windings **124T** which include a plurality of conductive turns surrounding different one or more of the magnetic core sections **122T.** To this end, in various embodiments, the electric channel **128** between the electric contacts (shown as **123** in **Figs. 1A** to **1E****),** may be printed as a single conductive path passing between the pair of electric contacts **123** and shaped to form the winding sets **128A** to **128D** along a single conductive path, or the electric channel **128** between the electric contacts may be printed to split into several (e.g. 4) electric sub channels (e.g. which may be connected to one another e.g. in series) and each forming a plurality of windings/turns of the one or more winding sets **128A** to **128D.**

With reference back to **Figs. 1A to 1E****,** it should be noted that the magnetic channel **122L** of the longitudinally oriented coils **120L** may be in the form of a rod (e.g. straight core) directed along the magnetic axis **L** of the coil **120L** (i.e. along the printing/longitudinal direction **Z)** as illustrated in the **Figs. 1A****,** **1B** **and** **1D****.** In this case printing the magnetic channel **129** and/or the magnetic core **122L** of the longitudinally oriented coils **120L** with magnetic material **M1** is not strictly necessary since with windings turns printed planarly within the printed layers of the sensor **100** they are inherently oriented to sense/capture magnetic flux propagating along the magnetic axis **L** of the longitudinally oriented coil **120L** (as the printing direction is substantially parallel to the magnetic axes **L** of the longitudinal coils **120L).** Accordingly in general, in case the magnetic channel/core **129/122L** passing through the windings **124L** of the longitudinally oriented coils **120L** is in the form of a straight rod, it may generally be printed with either magnetic material **M1** and/or with nonmagnetic material, such as with ceramic or glass-ceramic, or it may remain unprinted/blank (e.g. air channel). Nonetheless, typically in order to facilitate improved sensitivity of the longitudinally oriented coils **120L** (e.g. to collect and channel more magnetic flux through its windings **124L)** the magnetic channel **129** of the longitudinally oriented coils **120L,** and in particular the core section thereof passing through the windings **124L,** is typically printed with magnetic material **M3** in like manner as described with reference to the transversely oriented coils **120T.** Moreover, optionally as illustrated in **Fig. 1B** **and** **1D****,** in order to further improve the sensitivity of the longitudinally oriented coils **120L** to magnetic flux from the direction of the magnetic axis **L,** the magnetic channel **122L** is printed in some embodiments with respective pair of flux collectors **126L** arranged from opposite ends thereof. The optional pair of flux collectors **126L** are configured with respective relatively wide/large-area facets/surfaces **126U** and **126D** facing (e.g. perpendicular-to) the magnetic axis **L** of the longitudinally oriented coil **120L;** e.g. with width/area substantially larger than the characteristic width/cross-section-area of the magnetic core section **122L** between them (e.g. in order to channel more magnetic flux through the coil and thereby improve its sensitivity). Optionally the flux collectors **126L** are tapered towards the core section **122L** of the magnetic channel in order to facilitate efficient flux channeling between the relatively wide flux collection facets/surfaces **126U** and **126D** and the narrower magnetic core section **122T).**

It should be understood that the shape of the magnetic channel **122L** of the longitudinally oriented coils **120L** is not necessarily in the form of a straight rod. For instance in some embodiments where the magnetic channel **122L** of the longitudinally oriented coils **120L** is printed with magnetic material **M3** that can efficiently channel the magnetic flux, the magnetic channel **122L** may also be 3D printed to follow a curved/folded path within the monolithic body **110** of the sensor **100** in order to facilitate channeling the magnetic flux through higher count of turns of the conductive windings **124L** surrounding it, and or channeling it to pass multiple times through the winding' turns. This may provide higher sensitivity of the longitudinally oriented coils **120L.**

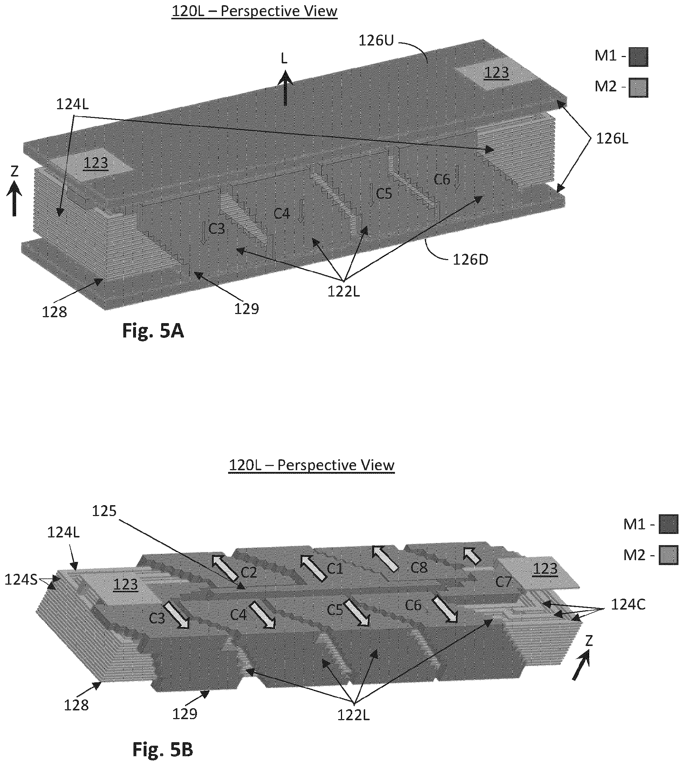

For instance, magnetic channel **122L** of the longitudinally oriented coils **120L** may be curved/folded to follow/form a helical-like path/shape or a meander-like path/shape similar to those illustrated above for the transversely oriented coils **120T** with reference to **Figs.2A to 4B** (e.g. only with the flux collectors arranged such that their flux collection surfaces are directed perpendicular to the magnetic axis **L** of the longitudinally oriented coil so as to collect magnetic flux from that direction). Alternatively, or additionally in some implementations the magnetic channel **129** of the longitudinally oriented coils **120L** may be curved/folded to follow/form a coiled-like path/shape (e.g. such that it is wrapped about the conductive windings **124L)** as illustrated for instance in **Figs. 5A and 5B****.**

To this end, **Figs. 5A and 5B** are schematic illustrations exemplifying a longitudinally oriented coil **120L** 3D printed in the sensor **100** such that its magnetic channel **129** is curved/folded to form a coiled-like path/shape of its magnetic core **122L,** wrapped about the conductive windings **124L.** Like reference numerals are used in **Figs. 5A and 5B** to designate elements/sections/parts of similar functionality as in the longitudinally oriented coils described above with reference to **Figs. 1A to 1E****.**

**Fig. 5A** depicts a perspective views of the coil **120L** including the flux collectors **126L,** and **Fig. 5B** depicts another perspective view of the coil **120L** without showing the flux collectors **126L,** so that the internal configuration of the coiled magnetic core **122L** and its wrapping about the conductive windings **124L** is visible in the figure. To this end, magnetic flux collected by one of the flux collectors **126L** is channeled by the magnetic channel **129** to circumference the conductive turns of the windings **124T,** typically for several times. For instance, magnetic flux collected from the top flux collection surface **126U** illustrated in **Fig. 5A** is channeled through the magnetic channel **129** from the region **125** (being the connection between the flux collector to the coiled magnetic core **122L)** of the magnetic channel **129** to follow sequentially through coiled sections **C1** to **C8** and then exit the coil though the bottom flux collection surface **126D.** The direction of the flux flow through the coiled core sections **C1** to **C8** of the magnetic core **122L** is depicted. To this end, the coiled core sections **C1** to **C8** are wrapped/winded about the turns of the conductive windings **124T** (e.g. intertwined therewith) in a manner that induces voltage/current in the conductive windings **124T** in response to magnetic flux propagating through the coiled core sections **C1** to **C8,** and thereby voltage at the electric contact **123.**

It should be noted that in this non-limiting example, in order to provide further improved sensitivity of the coil, the conductive windings include plurality of turns **124S** stacked along the printing direction **Z** in a plurality of printing layers (e.g. with relatively small spacing between them along the printing direction **Z),** wherein in at least some of the printing layers the multiple turns **124C** are printed concentrically in order to provide further improved sensitivity of the coil.

To this end, **Figs. 1A** to **5B** described above, schematically illustrate various non-limiting example of a multi-axis 3D printed magnetic field sensor **100** including at least one coil **120L** whose magnetic axis **L** is oriented longitudinally along the direction of printing **Z** of the sensor (the direction along which printed layers are deposited one on top the other), as well as at least one coil **120T** whose magnetic axis **T** is oriented transversely (e.g. perpendicularly) to the printing direction of printing **Z.**

Preferably, in both the longitudinally oriented coil(s) **120L** and the transversely oriented coil(s) **120T** turns of the conductive windings **124** are 3D printed within the printing layers in order to exploit the smaller pitch between printing layers, which is smaller than the minimal feature size/width within the layer, for packing/printing a higher count of conductive winding turns in the coils and facilitate high sensitivity of the sensor (i.e. facilitate high induced voltage in response to magnetic field sensing). To achieve that, for the transversely oriented coil(s) **120T** whose magnetic axis **T** is generally perpendicular/intersecting the printing direction, the magnetic channel **129** thereof is printed with a pair of flux collectors **126T** facing the magnetic axis **T** to collect flux propagating therefrom, and is further printed with turn(s)/fold(s)/curve(s) **127** between the pair of flux collectors **126T** so that a section thereof extends along the printing direction **Z** and passes through the conductive windings **124TL** to serve as a magnetic core **122T** thereof, which channels the magnetic flux therethrough. In non-limiting embodiments specifically exemplified herein the magnetic channel **129** of the transversely oriented coil(s) **120T** is exemplified with a stretched **"Z"** like shape **(****Figs. 1A****,** **1C** **and** **1E****),** with helical shapes **(****Figs. 2A to 3B****)** and/or with meander-like shape **(****Figs. 4A and 4B****).** Yet it should be understood and would be readily appreciated by those versed in the art that the transversely oriented coil(s) **120T** according to the present invention can be formed with other magnetic channel **129** shapes as well, for example with proper adaptations it may be implemented with a coiled shape of the core, as exemplified in **Figs. 5A and 5B** for the longitudinal coils **120T.** As for the longitudinally oriented coil(s) **120L,** their magnetic channels may path through the planarly/in-later printed windings turns **124T** of the coils while being formed as a straight rod-like core along the printing direction **Z** (e.g. as in **Figs. 1A****,** **1B** **and** **1D****).** Nonetheless, in some embodiments it may be preferable to print also the magnetic channel of the longitudinally oriented coil(s) **120L** with more intricate shapes such as the coiled core shape illustrated in **Figs. 5A** and **5B****.** In this regard it should be understood and would be readily appreciated by those versed in the art that the longitudinally oriented coil(s) **120L** according to the present invention can be formed with other magnetic channel **129** shapes as well, for example with proper adaptations it may be implemented with a meander or helical shape of the core, as exemplified in **Figs. 2A to 4B****.**

Furthermore, it should be noted that some optional properties of the 3D printed coils, which were illustrated only in some embodiments described above, can be readily implemented in other configurations of magnetic sensors of the invention without departing from the invention. For instance, as would be appreciated by those versed in the art, the following optional properties/features may be implemented in various embodiments of the invention and not only in those they were depicted above:
- the splitting of the magnetic channel **129** to form a plurality of magnetic subchannels thereof between the flux collectors as illustrated in **Figs. 3A to 3C****;**
- the splitting of the electric channel to form multiple conductive paths and optionally multiple winding sets, as illustrated in **Figs. 3A to 3C****;** and
- the formation of a conductive windings with both stacking of turns in multiple layers as well as forming concentric turns in each, or at least some of the layers.

Typically, one or more of the coils **120** of the sensor **100,** or all of them, are designed with an open magnetic circuit configuration that is suitable for accurate sensing of magnetic fields, and in particular suitable for magnetic field bases position sensing in medical applications/devices such as in catheters. In this context the phrase *open magnetic circuit configuration* should be understood herein as relating to coil structures with an open-ended magnetic channel that poses substantially no shielding or shunting of external magnetic fields enabling the external magnetic field to pass through the coil's magnetic core.

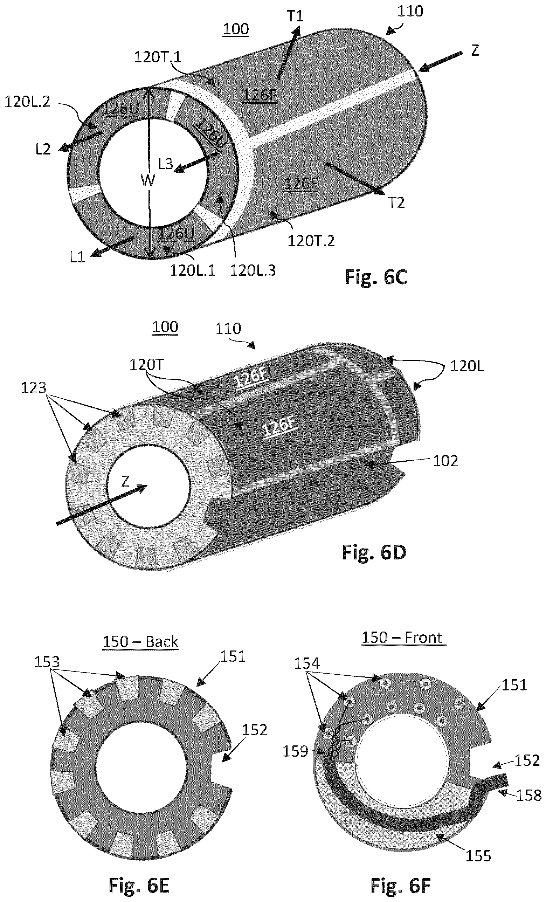

As indicated above the 3D printed magnetic field sensor **100** of the present invention as described above, particularly when printed with the coils **120** having open magnetic circuit configurations, may be suited for use as position and/or force sensors in medical instrument/device requiring the same. Reference is made together to **Figs. 6A to 6C** exemplifying a medical instrument **1000** (e.g., probe/catheter) according to an embodiment of the present invention being fitted with a magnetic field sensor **100** configured and operable according to an embodiment of the invention as a *Five Axes* position sensor (3D spatial position, pitch and yaw). **Fig. 6A** is a side-view of the medical instrument **1000** fitted with the magnetic-field (position) sensor **100** and **Fig. 6B** **and** **6C** are two perspective views exemplifying internal structure **(6B)** and the flux collectors' configuration **(6C)** of a magnetic field sensor **100** configured/fabricated according to an embodiment of the invention.

To this end, with reference to **Fig. 6A****,** the medical instrument **1000** includes in this non-limiting example a housing **H** having a longitudinal axis **Z** with a five-axes magnetic field-based position sensor **100** furnished therein. In this example the magnetic field-based position sensor **100** is furnished such that the longitudinal axis **Z** of the housing is along the same direction as the printing direction of the magnetic field-based position sensor **100.** In this embodiment the magnetic field-based position sensor **100** is configured to sense magnetic fields from along 5 Axes, including three spaced apart longitudinal axes **L1** to **L3** which are parallel to the longitudinal axis **Z** of the housing and two transversal axes **T1** and **T2** which are not parallel to one another and not parallel to the longitudinal axis **Z** of the housing.

The medical instrument **1000** is associated with one or more magnetic field external sources **MF_{L}** which generates reference magnetic fields whose magnitude and/or phase provide a frame of reference for determining the position and/or orientation of the medical instrument **1000** relative thereto. In other words, the reference magnetic fields generated by the magnetic field external sources **MF_{L}** can be measured along three axes by the magnetic field-based position sensor **100** of the medical instrument **100** to determine the 3D spatial location and/or orientation of the medical instrument **1000** relative to the external magnetic field sources **MF_{L}.** To achieve that, in this embodiment the magnetic field-based position sensor **100** includes at least one longitudinally oriented coil **120L.1** and at least two transversely oriented coils **120T.1** and **120T.2** whose respective magnetic axes **L1, T1** and **T2** are not parallel to one another and span three dimensions. The at least one longitudinally oriented coil **120L.1** and at least two transversely oriented coils **120T.1** and **120T.2** are typically printed with an open magnetic circuit configuration in order to sense the magnetic field generated by the external magnetic field source **MF_{L}** with improved sensitivity. Accordingly, the three coils **120L.1, 120T.1** and **120T.2** facilitate measurement of different vector components of the magnetic field generated by the external magnetic field source **MF_{L},** and by such measurement, as will be readily be appreciated by those versed in the art, facilitated determination of a location and orientation of the medical instrument **1000** to the reference frame defined by the magnetic field of the external magnetic field source **MF_{L}.**

In an embodiment, the medical instrument's housing **H** includes a main section **M** and a tip section **T** coupled to one another via a banding coupler **J,** such as a spring or joint, that is configured to facilitate bending the orientation of the tip section **T** relative to the main section **M** relative to the longitudinal axis **Z** of the housing **H** under applied force **F** (e.g. a force which may be applied when tip section **T** of the medical instrument is in contact with a body tissue of a patient).

The medical instrument **1000** includes therein a magnetic field source **MFo** which provide reference magnetic field that facilitates measurement of the degree of bending between the tip **T** and the main section **M** by the magnetic field-based position sensor **100.** Typically, the bending degree is indicative of a magnitude and/or direction of the force **F** applied to the tip section **T** (e.g. applied between the tip and a body tissue of a patient). In order to measure the magnitude and direction of a force **F** applied by the tip **T** to a tissue, the medical instrument **1000** includes an magnetic field source **MFo** (e.g., magnetic field generating coil with associated power delivery circuitry) and the magnetic-field-based positioning sensor **100,** such that they are arranged therein from opposite sides of the joint **J** (typically the internal magnetic field source **MFo** is arranged in the tip section **T** and the magnetic-field-based positioning sensor **100** is arranged at the main section **M,** although opposite arrangement may also be applicable). The internal magnetic field source **MFo** is configured and arranged in the housing **H** (e.g. in the tip **T)** for producing a magnetic field with flux lines/axis generally along the longitudinal axis **Z** of the housing **H.** The magnetic-field-based positioning sensor **100** includes a monolithic body **110** with a plurality of at least three longitudinally oriented coils **120.1, 120.2** and **120.3** such as those exemplified above with reference to **Fig. 1A to 5B** above, which are 3D printed in the monolithic body according to the technique of the present invention with their respective magnetic axes **L1 to L3** parallel to the longitudinal axis **Z** of the housing (being also the printing direction of the sensor **100).** The three longitudinally oriented coils **120.1, 120.2** and **120.3** are typically printed with an open magnetic circuit configuration to and adapted to sense the magnetic field generated by the internal magnetic field source **MF_{O}** with improved sensitivity. As will be appreciated by those versed in the art, since the magnetic axes **L1 to L3** of the three longitudinally oriented coils **120.1, 120.2** and **120.3** are collinear/parallel to one another and parallel to the longitudinal axis **L** of the housing **H** and not laying on a common plane (not co-planar), the magnetic fields measured thereby from the internal magnetic field source **MFo** (e.g. its measured amplitude by each of the coils) is indicative of the relative orientation about two orientation axes (e.g. pitch and yaw) between the magnetic field position sensor **100** and the internal magnetic field source **MFo.** To this end, having considered the technique of the present invention person of ordinary skill in the art will readily appreciate how the orientation of the sensor **100** relative to the internal magnetic field source **MFo** may be determined based on measurements of the magnetic field generated by the source **MFo** made by the three longitudinally oriented coils **120.1, 120.2** and **120.3.** Moreover, a person of ordinary skill in the art will readily appreciate how the force vector **F** applied to the tissue may be determined based on this measured orientation of the sensor **100** and the properties (e.g., spring constant) of the banding coupler **J.**

Thus, in this non-limiting example, as the magnetic field sensor **100** is designated to sense both a location and orientation thereof relative to the external magnetic field source **MF_{L}** and an orientation thereof relative to the internal magnetic field source **MF_{O}.** Accordingly, it is configured as a *Five Axes* magnetic field sensor capable of measuring magnetic fields from align three longitudinal axes **L1** to **L3** and two transversal axes **T1 and T2.** It should be however noted that present invention is not limited to the five-axes configuration of the magnetic-field/positioning sensor **100,** and may also be used to implement magnetic-field/positioning sensor **100** with two-, three-, or four- sensing axes, and/or with any other desired number of sensing axes. For instance:
- A three magnetic axes sensor - for measurement of 3D location/orientation of a medical device relative to an external magnetic field (3 axis sensor);
- A two magnetic axes sensor - for measurement of 2D location/orientation of a medical device relative to an external magnetic field;
- A two magnetic axes sensor - for measurement one angle of orientation (e.g. either pitch or yaw) of the sensor relative to an internal magnetic field;
- A three or four magnetic axes sensor - for respectively measuring one angle of orientation (e.g. either pitch or yaw) of the sensor relative to an internal magnetic field as well as the 2D or 3D location/orientation of a medical device relative to an external magnetic field; and
- A five magnetic axes sensor as in the present example - for measuring two angles of orientation (e.g. pitch and yaw) of the sensor relative to an internal magnetic field as well as the 3D location/orientation of a medical device relative to an external magnetic field.
In this regard, it should be noted that in view of the description herein a person of ordinary skill in the art, after knowing the invention, will readily appreciate how to fabricate a magnetic field sensor by 3D printing with any suitable number of coils and various configurations/directions of their magnetic axes relative to the printing direction **Z** to facilitate accurate/sensitive measurement of the sensor's location and/or orientation relative to one or more magnetic field sources in one, two and/or three dimensions.

A perspective view illustrating in more details the configuration of a five-axes magnetic field sensor **100** according to an embodiment of the present invention, which is incorporated in the medical device **1000** described above, is shown in **Figs. 6B** **and** **6C****.** **Fig. 6B** is a perspective view of the magnetic field sensor **100** depicting the internal construction/coils of the five-axes magnetic field sensor **100** with the monolithic body **110** thereof depicted only by dashed-dotted contour lines (the printed bulk material **M3** thereof is not illustrated so as not to obscure the configuration of the 3D printed sensor's coils **120).** **Fig. 6C** is another perspective view of the five-axes magnetic field sensor **100** illustrating the arrangement of some of the coils' flux collectors **126** of the sensor **100.** The magnetic field sensor **100** in this example is fabricated by the 3D printing technique of the invention as described above with reference to **Figs. 1A to 5B** and includes three longitudinally oriented coils **120L.1** to **120L.3** with respective parallel magnetic axes **L1** to **L3** directed along the longitudinal/printing direction **Z** and two transversely oriented coils **120T.1 and 120T.2** with respective non-parallel magnetic axes **T1** and **T2** directed in two transvers direction.

The three longitudinally oriented coils **120L** are coaligned such that their respective magnetic axes are parallel to one another. This arrangement of longitudinally oriented coils **120L** facilitate utilizing the magnetic signals sensed/obtained thereby to determine two angles of orientation of the magnetic field sensor **100** relative to the internal magnetic field source **MFo** located in-front of the magnetic field sensor **100** with respect to the longitudinal direction **Z.** Additionally, as the magnetic axes, **T1** and **T2,** of the two transversely oriented coils **120T** and the magnetic axis e.g. **L1** of at least one e.g. **120L** of longitudinally oriented coils span 3D coordinates, the magnetic signals sensed/obtained from these two transversely oriented coils **120T** and the at least one longitudinally oriented coil **120L** may be utilized/processed to determine the position (e.g. location and/or orientation) of the sensor relative to the reference coordinate frame provided by the magnetic fields of the external magnetic field source(s) **MF_{L}.**

In this example the magnetic field sensor **100** has an elongated tubular/cylindrical shape, elongated about the longitudinal/printing axis **Z** with width/diameter **W** sufficiently small to fit within the housing **H** of the medical instrument **1000.** For instance, the inner diameter the housing **H** and accordingly the width/diameter **W** of the magnetic field sensor **100** may be in the order of about 2 millimeters.

In some embodiments in order to facilitate compact arrangement of the coils **120L.1** to **120L.3** and **120T.1** and **120T.2** within the narrow monolithic body **110,** the three longitudinally oriented coils **120L.1** to **120L.3** are arranged in respective three angular sectors about a longitudinal/printing axis **Z** and the two transversely oriented coils **120T.1** and **120T.2** are arranged in respective two angular sectors about that axis. Preferably, in some embodiments the three longitudinally oriented coils **120L.1** to **120L.3** are equally spaced (e.g. forming an equilateral triangle) so as to obtain equally "weighted" magnetic field signals from the internal magnetic field source **MFo.** For instance, in the present non-limiting example the three longitudinally oriented coils **120L.1** to **120L.3** are equally angularly spaced from one another by 120°. The transversely oriented coils **120T.1** and **120T.2,** e.g. their magnetic axes **T1** and **T2,** are typically angularly spaced as well as oriented relative to one another, with respect to the longitudinal/printing axis **Z,** by an angle that is preferably relatively close to 90° (e.g. within a range of few tens of degrees from 90°) so that that the magnetic signals readout therefrom can be used/process to identify/determine two perpendicular vector components of the magnetic fields sensed thereby with high SNR (e.g. not having too much overlap between the vector components of the magnetic fields the individually sense). Yet, typically the relative angular spacing between the transversely oriented coils **120T.1** and **120T.2** is further optimized to facilitate compact arrangement of the sensor's coils **120** in the monolithic body, or other features thereof.

For instance, in the embodiment illustrated for example in **Fig. 6B****,** transversely oriented coils **120T.1** and **120T.2** and their magnetic axes **T1 and T2** are angularly spaced/oriented relative to one another about the longitudinal/printing axis **Z,** by an angle of **120°** which in the case (with this configuration/sizes/shapes of the coils) facilitates a compact arrangement of coils with relatively high sensitivity within the restricted maximal dimensions of the sensor **100** to fit in the medical instrument **100.**

To this end, in some embodiments, compact dimensions of the sensor, as well as high quality position signal sensing (e.g. sensing of substantially equally weighted vector components of the desired magnetic fields, is achieved by arrangement of the five coils, **120L.1** to **120L.3** and **120T.1** and **120T.2** in five respective angular sector that are in the same level/height with respect to the Z axis). Alternatively in some embodiments (not specifically illustrated in the figure) compact dimensions of the sensor as well as high quality position signal sensing, are optimally obtained by an arrangement of the three longitudinally oriented coils **120L.1** to **120L.3** at angular sectors in one level/height with respect to the longitudinal/printing **Z** axis, and the two transversely oriented coils **120T.1** and **120T.2** in two angular sectors of another level/height with respect to the longitudinal/printing **Z** axis.

As illustrated in this non-limiting example, the magnetic field sensor **100** has an annular shape with a hole **115** fabricated (not being printed) through its monolithic body **115** along the longitudinal axis **Z** in order to enable some components of the medical device **100** (such as catheter's electrical/signal lines and/or fluid lines) to be passed therethrough. To this end, with such an elongated shape of the sensor **100** the maximal distance **D** between the pair of opposite flux collectors **126T** is severely limited. For example in sensors having annular shapes with width **W** of about 2mm with each of the transversely oriented coils **120T** including a flux collector **126T** near an external facet of the annular shape and a flux collector **126T** located near an internal facet of the annular shape facing the hole **115,** as illustrated in the figure, the maximal distance **D** between the opposite flux collectors **126T** along the magnetic axis **T** of the transversely oriented coils **120T** is restricted to substantially less than 1mm (e.g. typically to only about 0.5 mm considering the hole **115** has a diameter of typically about 1 mm or less. Therefore, with such short distance along the magnetic axis **T** of the transversely oriented coils, it is not practical with current conventional 3D printing technologies, to fabricate transversely oriented coils **120T** with a straight/rod magnetic core that would provide a sufficiently sensitivity suitable for medical device positioning. This issue is overcome by the technique of the present invention, by printing the magnetic core of the transversely oriented coils **120T** such that it includes a magnetic channel **122T** between the flux collectors 126T that curves/folds within the monolithic body **110** of the sensor **100** such that it has an extended length (e.g. greater than the distance between the flux collectors **126T),** thereby facilitating 3D printing of higher count of conductive windings **124T** about the magnetic channel **122T** (as compared to a case of a shorted rod core) which in turn improves the sensitivity of the transversely oriented coils **120T** of the sensor despite its elongated shape.

With reference **6D** to **6F** an embodiment of the sensor **100** is illustrated configured to facilitate a robust reliable and cost-effective connection of signal lines **159** to the sensor's coils **120** before/during the fitting of the sensor's monolithic body **110** to the medical instrument **1000.** In this embodiment the sensor **100** includes a signal connector **150** exemplified as optional in **Fig. 6A** and illustrated in details in **Figs. 6E and 6F****,** and the 3D printed monolithic body thereof **110** with the plurality of coils **120** 3D printed therein (including transversely and longitudinally oriented coils **120T** and **120L).** As illustrated in **Fig. 6D** the electric contacts/terminals **123** of the coils **120** are exposed from the monolithic body **110** at a surface thereof, being typically a planar surface. In this example there are 10 electric contacts/terminals **123,** two per each of the five coils **120,** which are arranged in a predetermined arrangement. The signal connector **150** whose back and front sides are respectively illustrated in **Figs. 6E and 6F** includes a printed circuit board (PCB) **151** with a complementary arrangement of electric contact pads **153** from its back side **(Fig. 3E)** matching the arrangement of the electric contacts/terminals **123** in the monolithic body **110.** Each of the electric contact pads **153** at the back side **(Fig. 3F)** of the signal connector **150** is electrically coupled, e.g. by conductive vias and/or redistribution lines (RDLs) in the PCB **151,** to soldering contacts **154** at the front side of the PCB **151.** A signal cable **158,** including a plurality of conductive signal lines **159,** typically shielded signal lines such as twisted pairs, is electrically connected to the soldering contacts **154,** typically by soldering/wire-bonding. More specifically in this example there are 5 twisted pairs (only two are illustrated in the figure for clarity) each of which being soldered to a respective pair of the soldering contacts **154** which are electrically coupled (via the RDLS/vias) to electric contact pads **153** that are arranged in the back side of the signal connector **150** to contact the pair of electric contacts/terminals **123** of one of the coils **120** in the monolithic body **110.**

In some embodiments optionally the signal connector **150** includes at its front side a fixation area **155,** to which the signal cable **158** may be attached (e.g. pasted) to improve the robustness and reliability of the electrical connections thereof to the soldering contacts **154** of the signal connector **150** (e.g. such that they reliably endure stresses/tensions which might be applied thereto during the fitting of the sensor **100** within the narrow space of the medical instrument **1000).**

To this end, during the fitting of the sensor **100** in the medical instrument **1000,** e.g. before or after the monolithic body **110** is inserted to the instrument **1000,** the signal connector **150** may be attached to the monolithic body **110** such that the electric contact pads **153** at the back side of the signal connector **150** are electrically coupled to the complementary arrangement of electric contacts/terminals **123** of one of the coils **120** in the monolithic body **110** (as will be appreciated by those versed in the art this may be performed reliably using soldering for instance using flip-chip bonding).

In some embodiments, as exemplified for instance in **Figs. 6D to 6F****,** the monolithic body **110** of the sensor **100** and the signal connector **150** respectively include complementary trench **102** and notch **152** arranged such that they are aligned when the signal connector **150** properly coupled to the monolithic body **110** with the electric contact pads **153** properly contact the respective electric contacts/terminals **123.** Considering the miniature size of the sensor **100** in some implementations, the trench **102** and notch **152** facilitate accurate and easy alignment between the monolithic body **110** of the sensor **100** and the signal connector **150** when they are fitted/coupled together such that the electric contact pads **153** are properly coupled to the respective electric contacts/terminals **123.** Alternatively, or additionally, in some embodiments the trench **102** and notch **152** provide a pathway for passing the signal/power lines towards the tip section of the medical instrument **1000** (for example delivering power to the internal magnetic field source **MFo)** through the narrow housing **H** of the medical instrument **1000,** when the sensor **100 is** furnished therein.

It should be noted that in some implementations the of the magnetic-field/positioning sensor **100** described with respect to all the embodiments above, the electrically conductive material **M2,** which is used in 3D printing of the electric channels **128** of the coils **120** includes Silver. Alternatively, or additionally in some embodiments the electrically conductive material **M2** may include Copper, Silver, Palladium, and/or Silver-Palladium alloy. The magnetic material **M1**, which is used for 3D printing the magnetic channels **129** of the coils **120** (i.e. their magnetic cores **122** and flux collectors **126)** may generally include Ceramic-Ferrite material and/or Metal Material. In some embodiments the magnetic material **M1** is a soft magnetic material having relative permeability µᵣ of at least 100 or above. Preferably the magnetic material **M1**, particularly that used for printing the magnetic channels **129** of the transversely oriented coils **120T,** is a material having relative permeability µᵣ in the order of 5000 or above (in some implementations the folded/curved magnetic channel has relatively small cross-section, and therefore a relatively high permeability µᵣ in the order of 5000 or above is needed in order to control/facilitate the funneling of the magnetic field flux through, with sufficiently small losses). This is in order to facilitate efficient channeling of magnetic flux through the folded/curved magnetic channel of the transversely oriented coils **120T.** The non-magnetic dielectric material **M3** (also referred to herein as the bulk material) may for instance include ceramic and/or glass-ceramic. Preferably in some embodiments the conductive material **M2** of the conductive channel(s) **128** is printed such that is fully embedded within the bulk of the monolithic body **110** so as to be isolated from environmental conditions by which it might be degraded. In some embodiments optionally also the electric contacts/terminals of the conductive channel(s) are 3D printed embedded within the bulk, and only exposed to the outside (e.g. by polishing) only after the 3D printed sensor is sintered/fired.

As indicated above the magnetic-field/position sensor **100** of the invention, as exemplified in all the above embodiments is fabricated by 3D printing of at least three different 3D-printable materials **M1 to M3.** **Fig. 7** is a flow diagram of a method **200** which is used according to some embodiments of the present invention, to fabricate the magnetic-field sensor **100** described above by 3D-printing technology.

In operation **210** of the method **200,** a model of one or more magnetic field sensors **100** of the invention, such as those described above with reference to the embodiments in any one of **Figs. 1A to 6F****,** which are to be 3D-printed, are provided. The model is indicative 3D spatial distribution (voxel maps) of at least three materials, **M1 to M3,** which are to be 3D-printed to fabricate the magnetic field sensors **100** of the present invention. Particularly the model is indicative of the respective spatial distributions of the three materials **M1 to M3** that should be 3D printed to form a magnetic field sensor **100** according to the invention. More specifically the spatial distributions of the three materials **M1 to M3** is such that the **3D** printing yields a magnetic field sensor **100** having monolithic body **110** with one or more longitudinally oriented coils **120L** and one or more transversely oriented coils **120T** printed therein.

Typically, the multi-material 3D-printing used operates by successive printing of layers, one on top the other, along the printing direction **Z.** Each layer may include one, two or all three materials. The spatial distributions of the three materials **M1 to M3** in the model used for the printing is such that the printing results with a plurality of coils **120** defined by the distribution of the three materials **M1 to M3** within the monolithic body **110,** and such that the bulk of the monolithic body is 3D printed with the non-magnetic non-conductive material **M1** and the plurality of coils **120** therein include:
- one or more longitudinally oriented coils **120L** whose respective magnetic axes **(L)** are oriented substantially parallel to the printing direction **Z** and each includes conductive windings **124L** including an arrangement of conductive turns 3D printed planarly within a plurality of the 3D printed layer, with the conductive material **M2;** and
- one or more transversely oriented coils **120T** whose respective magnetic axes **(T)** are oriented substantially transversely/perpendicularly to the printing direction **Z.** Each transversely oriented coil **120T** includes a magnetic channel **129** 3D printed with magnetic material **M1**, such that the magnetic channel **129** including at least a pair of opposite flux collectors **126T** with respective relatively large facets perpendicular to its transversely oriented magnetic axis **(T).** The magnetic channel **129** of the transversely oriented coil **120T** is printed with one or more curves/ folds **127** within the monolithic body **110** such that one or more sections **122T** thereof extends substantially along the printing direction **Z** to serve as magnetic core of the coil, and the transversely oriented coils **120T** includes conductive windings **124T** including an arrangement of conductive turns 3D printed planarly within a plurality of the 3D printed layer, with the conductive material **M2,** such that they surround the magnetic core **122T.**
The spatial distributions of the three materials **M1 to M3** in the model may further define other features of the sensor **100** described above, for instance: magnetic channel may be fabricated also for the longitudinally oriented coils **129;** the magnetic channels **129** of the longitudinally and/or transversely oriented coils may be formed various further more intricate shapes as described for instance in **Figs. 2A to 5B****;** the conductive windings of the coils may generally be 3D printed as a part of an electric channel **128** terminating by two or more electric contacts/terminals **123** at a surface of the monolithic body 110; and the monolithic body **110** may be formed with an elongated/tubular shape and/or with a cylindrical and/or annular shape with a hole **115** therein, or other body geometry that fits into the particular catheter.

In operation **220** of method **200,** a multi-material 3D-printing is applied to print the one or more magnetic field sensors **100** based on the spatial distributions of the three or more materials **M1 to M3** in the model. The multi-material 3D-printing may be carried out for example by utilizing a photopolymerization 3D-printing technique to print layers that are composed of a combination of several materials having different electromagnetic properties (e.g., two three or optionally more than three materials with different electromagnetic properties may be printed in each layer). In a typical example, 3D printing of the magnetic field sensor **100** described above involves printing of spatial distributions of at least three materials of different electromagnetic properties including: electrically conductive material **M2,** for 3D printing of the electric channels **128** of the coils **120;** magnetic material **M1**, for 3D printing the magnetic channels **129** of the coils **120;** and non-magnetic dielectric material **M3** for 3D printing the bulk of the monolithic body **110** of the sensor **100.**

To this end in various embodiments the electrically conductive material **M2** may include for example Silver, and/or Copper, and/or Palladium and/or Silver-Palladium alloy. The magnetic material **M1** may include for example Ceramic-Ferrite material and/or Metal Material. In some embodiments the magnetic material **M1** is a soft magnetic material having relative permeability µᵣ of at least 100 or above. Preferably the magnetic material **M1**, particularly that used for printing the magnetic channels **129** of the transversely oriented coils **120T,** is a material having relative permeability µᵣ in the order of 5000 or above in order to facilitate efficient channeling of magnetic flux through the folds/curves **127** thereof. The non-magnetic dielectric material **M3** (also referred to herein as the bulk material) may for instance include ceramic and/or glass-ceramic.

The 3D printing of the sensor **100** may be implemented for example based on the principles of Vat Photopolymerization, utilizing for instance stereolithography (SLA) or digital light processing (DLP) for the pattern implementation/projections. The invention is not limited to these specific technologies and may generally be implemented by other multi-material 3D printing technique as may be developed to be suitable for the fabrication of the electric components according to the invention.

With the technique of the invention the magnetic field sensors **100** with small feature sizes can be printed utilizing 3D printed layers with thicknesses in the scale down to microns (e.g., sintered layer thickness may be about 8-10 microns) and with in-layer features of characteristic sintered sizes in the scale of about 24-50 microns or less (this is considering that in layer pixel can be printed with pitch/widths of about 12-25 microns and considering the preference to print each important/functional feature with width of at least two pixels to avoid malfunctions due to single pixel misprints).

For carrying out the 3D printing, curable resins corresponding respectively to the at least three materials **M1, M2** and **M3** are provided. More specifically the curable resins used in the 3D printing of the magnetic field sensor **100** of the invention include for example the following:
- A magnetic material resin which includes particles of a soft magnetic material magnetic material **M1** suspended in curable polymer binder. The particles of the magnetic material **M1** may include for example particles of Ceramic-Ferrite material. It should be noted that alternatively in some embodiments the particles of the magnetic material **M1** may include particles of magnetic Metal Materials such as Iron alloy, iron-silicon alloy, iron-aluminum-silicon alloys, low-carbon steels, nickel-iron alloys, iron-cobalt alloys, ferrites, amorphous alloys, as well as in some cases Nickel-Cobalt Alloy (particularly in cases it has soft magnetic qualities). In embodiments where miniature coils are fabricated within the monolithic body **110** the particles of magnetic material used may be provided for example with characteristic sizes in the few micron to submicron scale, particularly in cases where the magnetic channels **129** of the coils manifest delicate features.
- A conductive material resin which includes particles of conductive material **M2** suspended in curable polymer binder. The particles of the conductive material **M2** may for instance include Silver material, Copper material and/or Silver-Palladium alloy. As specific choice of suitable conductive material may generally depend on the required temperatures of the sintering/firing process required to finalize the printing of the electric components and may thus depend on the types of magnetic and dielectric materials. In embodiments where miniature coils are fabricated within the monolithic body **110,** the particles of conductive material **M2** used in the printing may have characteristic sizes in the micron to submicron scale and as small as in the nanometer scale.
- A dielectric and non-magnetic material resin which includes particles of non-magnetic dielectric material **M3** suspended in curable polymer binder. The particles of the non-magnetic dielectric material typically include ceramic or glass-ceramic particles which can withstand the high sintering/firing temperatures required for sintering the printed magnetic and the conductive materials, **M1 and M2.** Also, here the size of the particles may be selected according to the minimal feature sizes that should be occupied by the dielectric material/bulk of the magnetic field sensor, and for fabrication of miniature coils may be selected for instance in the order of nanometer to micron scale.

In regards to the material selection in the above, it should be noted that although printing magnetic channel **129** with magnetic metal material is generally possible, in various embodiments printing the magnetic channel with an electrically insulating ceramic ferrite may be preferred. One reason for that is that the use ceramic ferrite facilitates achieving more compact arrangement of magnetic and electric channels, **129** and **128.** This is because soft magnetic metal materials (non-ceramic ferrite) that are suitable for use for the magnetic channel printing, are typically conductive. Therefore, a magnetic channel **129** printed with a non-ceramic ferrite, should be spaced from the electric channel **128,** at least at some places, in order to prevent short circuit, which might impact the achievable miniaturization of the magnetic field sensor **100 and/or** require more complex design thereof. Therefore, in some embodiments preferably ceramic ferrite material is used for the printing of the magnetic channels **129.**

Thus, utilizing the at least three curable resins described above, 3D printing of the magnetic field sensor **100** may be performed layer by layer along a printing direction **Z** utilizing for example photopolymerization multilateral 3D printing techniques such as SLA or DLP. The printing direction **Z** is typically selected such that it is substantially perpendicular to planes spanned by the turns of the conductive windings **124** of the coils **120.** To this end, as indicated above, the choice of the printing direction Z may have particular importance in the printing of miniature magnetic field sensors **100** because the lateral feature resolution of the printing in the X-Y plane is lower than the "vertical" resolution of the layers obtained along the printing direction **Z.**

To this end, typically the 3D printing of each layer of the sensor **100** typically includes the following (not necessarily in that order):
- Deposition/printing and curing the magnetic material **M1** at regions of the layer corresponding to the magnetic channels **129** of the coils **120;**
- Deposition/printing and curing the conductive material **M2** at regions of the layer corresponding to electric channel **128** of the coils **120;** and
- Deposition/printing and curing the non-electrically-conductive (dielectric) non-magnetic material **M3** to form the bulk of the monolithic body **110** of the sensor **100.**

It should be noted that in some embodiments the 3D printing is conducted such that the conductive material **M2** is fully embedded within the bulk of monolithic body **110** in order to protect the conductive material **M2** against degradation (e.g. oxidation) due to environmental conditions, particularly during the high temperature sintering **230** which follows layer by layer material deposition and curing.

Thus, by the consecutive printing and curing of successive layers in operation **220.** according to the spatial distribution of the materials in the sensor's model, one or more 3D structures of one or more sensors **100** each including at least one longitudinally oriented coil and at least one transversely oriented coil electric coil **220** are obtained.

Method **200** further includes operation **230** for sintering the one or more 3D structures of the one or more sensors **100** printed in operation **120.** The sintering is generally performed at temperature sufficient for burning or driving off polymers (binding polymers) from the one or more magnetic field sensors **100** as well as firing ceramic or glass ceramic materials such as **M3** and optionally **M1** which are used in the printing. To this end, after the sintering the material density of the printed ceramic and metal materials (e.g., **M1**, **M2** and **M3)** of the magnetic field sensors **100** approach 100%. For example, in embodiments where the conductive material **M2** mainly includes Silver, the sintering may be performed at temperatures below the Silver melting point (e.g., at temperatures not exceeding 961°C; preferable in this case the sintering temperature may be limited up to about 900C in order to achieve a stable sintering process). In other embodiments where the conductive material includes a Silver-Palladium alloy, the sintering may be performed at temperatures below the alloy melting point (the specific melting point depends on the ratio of silver and palladium in the alloy and depending on the composition the melting point is generally between the melting point of silver, 961°C, and that of palladium, 1,555°C). In another example, in embodiments where the conductive material **M2** includes Copper, the sintering may be performed in an Oxygen deprived environment (so as to avoid oxidation of the Copper), and at temperatures below the 1084°C Copper melting point.

In this connection, it should be noted that in some embodiments of the present invention the method **200** is carried out for 3D-printing magnetic-field/positioning sensors **100** in which miniature coils having features of micron scale size are embedded. For instance, the 3D printed layers may be printed with sintered thicknesses in a range of about 5 to 15 µm (e.g., 9µm), depending on the specific model design and 3D-Printing technology used. Accordingly, a pitch of the turns of the windings **124,** which may be in the order of twice the pitch of the 3D printed layers, may be within the range of about 10µm to 30µm sintered along the printing direction **Z** of the 3D printed sintered layers (e.g., typically layers' thicknesses are in the order of 9 µm and the turns' pitch between layers is of about 18 µm).

Further, optionally (depending on the specific printing technology used), the method **200** may include operation **240** for separating printed sensors **100** from the building/3D-printing platform (e.g., **201** shown in **Fig. 1E****).** Generally, depending on the type of building platform (e.g., and whether it can withstand the sintering **230),** the operation **240** may be performed before or after the sintering **230.** Indeed, in some embodiments of the invention, particularly where miniature/delicate sensors are printed, which may be damaged by carrying out their separation from the building platform before the sintering, a suitable building platform may be used which can withstand the temperatures of the sintering **230,** and the sensors' separation from the platform may be conducted post sintering so as to avoid/reduce damage to the sensors during the separation process.

In view of the above, method **200** of the present invention provides a novel and inventive technique for fabrication of magnetic-filed/positioning sensors **100** including a plurality of coils **120** as described above. The method **100** is suited for fabrication of miniature sensors **100** with features sizes as small as few microns, and may be used in mass production in order to simultaneously 3D print large pluralities of miniature such sensors **100** built in simultaneously on the same platform (e.g., typically between hundreds and several thousand may be printed using for instance a vat photopolymerization technology). Each magnetic-filed/positioning sensors **100** may have for example a ceramic/glass-ceramic body **110** embedding therein highly sensitive open magnetic circuit coils arranged in both transverse and longitudinal orientation with respect to the printing direction **Z.** The miniature magnetic-field/positioning sensors **100** may be fabricated with small dimensions, for example in the order of ~2mm in widths several millimeters in height with coils **120** therein having small dimensions of the order of ~0.5 mm to ~4 mm and providing high sensitivity/induced-voltage in response to magnetic fields, for example in the range -0.1 to 1 µV/(Gauss*Hz).

It should be noted that the illustrations of the magnetic-filed/positioning sensors **100** in **Figs. 1A** to **6F****,** are provided for illustrative purposes only, and are not necessarily drawn to scale or with proper proportions. For example, generally the count of winding in the coils **120** according to the present invention may be substantially higher than what is illustrated in the figure. Also, the specific shapes illustrated for the coils **120,** e.g., the specific shapes of their magnetic cores **122** windings **124** and flux collectors **126** are provided only as an example and may in practice have other shapes and/or aspect ratios than those illustrated, without departing from the scope of the present invention.

### Examples

Example 1. A magnetic field sensor including:
   a monolithic body with a plurality of coils defined therein by 3D printing of successive printed layers along a printing direction; the plurality of coils include:
   - one or more longitudinally oriented coils having respective first magnetic axes oriented substantially parallel to the printing direction; each of the longitudinally oriented coils includes a 3D printed first conductive channel forming an arrangement of first conductive windings with turns in planes of said printing layers; and
   - the plurality of coils further includes one or more transversely oriented coils with respective second magnetic axes transversely oriented relative to the printing direction.
Example 2. The magnetic field sensor according to Example 1, wherein each transversely oriented coil of the transversely oriented coils includes a magnetic channel 3D printed in the monolithic body such that the magnetic channel comprises at least a pair of opposite flux collectors having respective facets perpendicular to the second magnetic axis thereof and at least one section between them serving as a magnetic core of the transversely oriented coil; and a 3D printed second conductive channel forming an arrangement of second conductive windings 3D printed in the monolithic body to surround the at least one magnetic channel; and
   wherein the magnetic channel curves within the monolithic body such that the at least one section thereof, which serves as the magnetic core, extends along the printing direction, and the arrangement of second conductive windings includes a plurality of turns printed in planes of the printing layers to surround the at least one section serving as the magnetic core.
Example 3. The magnetic field sensor according to Example 2, wherein the monolithic body has an elongated shape along the printing direction with relatively short distance between the pair of opposite flux collectors, and wherein the curves of the magnetic channel within the monolithic body facilitate an extended length of the at least one section serving as the magnetic core, to accommodate higher count of turns of the second conductive windings about the extended length, thereby improving sensitivity of the transversely oriented coils despite the short distance between the pair of flux collectors.
Example 4. The magnetic field sensor according to any one of Examples 1 to 3, wherein the one or more longitudinally oriented coils include three longitudinally oriented coils of similar magnetic properties 3D printed such that they are coaligned and their respective first magnetic axes are parallel to one another, thereby enabling to utilize signals obtained from the three longitudinally oriented coils to determine two angles of orientation of the magnetic field sensor relative to a first magnetic field source located in-front of the magnetic field sensor along a general direction of the first magnetic axes; and
   wherein the one or more transversely oriented coils include two transversely oriented coils 3D printed such that their respective two transversely oriented magnetic axes are not parallel to one another and such that the two transversely oriented magnetic axes, together with a longitudinally oriented magnetic axis of at least one longitudinally oriented coil of the one or more longitudinally oriented coils, span 3D coordinates, thereby enabling to utilize signals obtained from the two transversely oriented coils and the at least one longitudinally oriented coil to determine a location and orientation of the sensor relative to one or more second magnetic field sources.
Example 5. The magnetic field sensor according to Example 4, wherein the at least three longitudinally oriented coils are arranged in respective three angular sectors about a longitudinal axis of the monolithic body being along the printing direction; and the at least two transversely oriented coils are arranged in respective two angular sectors about the longitudinal axis.
Example 6. The magnetic field sensor according to Example 5, wherein a least one of the following: (a) the three angular sectors and the two angular sectors are five coaligned sectors arranged about the longitudinal axis of the monolithic body in the same level relative to the longitudinal axis (same height with respect to the relative to the longitudinal axis); and (b) the three angular sectors and the two angular sectors are respectively arranged in different levels (different heights) with respect to the longitudinal axis of the monolithic body.
Example 7. The magnetic field sensor according to Example 5 or 6, wherein the monolithic body having a cylindrical shape about the longitudinal axis with characteristic width of about 2 millimeters to thereby enable fitting of the magnetic field sensor within a catheter.
Example 8. The magnetic field sensor according to Example 7, wherein the monolithic body has an annular shape with a hole passing therethrough along the longitudinal axis to facilitate passage of additional components (e.g. electric- and/or fluid-lines) of the catheter through the hole of the sensor; and wherein the a pair of opposite flux collectors of each of the transversely oriented coils include a flux collector arranged at an external facet of the annular shape and a flux collector located at a facet of the annular shape facing the hole.
Example 9. The magnetic field sensor according to any one of Examples 2 to 8, wherein the 3D printing provides a higher voxel resolution along the printing direction as compared to lateral voxel resolution within printing layers; and
   wherein the at least one section serving as the magnetic core extends substantially parallel to the printing direction and the turns of the second conductive windings that surround that at least one section are substantially perpendicular to the printing direction, thereby exploiting the higher voxel resolution along the printing direction to yield high density of the turns of the second conductive windings and thereby improved sensitivity of the transversely oriented coil.
Example 10. The magnetic field sensor according to any one of Examples 2 to 9, wherein the at least one magnetic channel curves in a helical-like or meander-like shape.
Example 11. The magnetic field sensor according to any one of Examples 2 to 9, wherein the at least one magnetic channel curves in a coil shape winded about the second conductive windings.
Example 12. The magnetic field sensor according to any one of Examples 2 to 11, wherein the facets of the flux collectors are wider than the at least one section serving as the magnetic core.
Example 13. The magnetic field sensor according to any one of Examples 2 to 11, wherein the magnetic channel is configured such that the transversely oriented coil has an open magnetic circuit configuration.
Example 14. The magnetic field sensor according to any one of Examples 1 to 13, wherein at least one longitudinally oriented coil of the one or more longitudinally oriented coils, includes a magnetic channel 3D printed in the monolithic body with a pair of opposite flux collectors having respective facets perpendicular to the first magnetic axis and at least one magnetic core section between the pair of opposite flux collectors; and wherein one or more of the following:
   - the pair of flux collectors of the longitudinally oriented coil are tapered towards the magnetic core section of the longitudinally oriented coil;
   - the magnetic core section of the longitudinally oriented coil is directed along said longitudinal direction in the form of a rod;
   - the magnetic channel of the longitudinally oriented coil curves in a coiled shape wrapped about the first conductive windings.
   - the magnetic channel of the longitudinally oriented coil curves within the monolithic body such that a greater length of the core section is surrounded by the first windings of the longitudinally oriented coil thereby facilitating higher output voltage and improved sensitivity of the longitudinally oriented coil;
   - the at least one longitudinally oriented coil is configured with an open magnetic circuit configuration; and
   - the pair of opposite flux collectors of the longitudinally oriented coil are arranged from opposite longitudinal sides of the monolithic body of the sensor.
Example 15. The magnetic field sensor according to any one of Examples 1 to 14, formed by 3D printing of at least three different 3D-printable materials including:
   - a magnetic material forming the magnetic channel;
   - a conductive material forming the conductive windings; and
   - non-magnetic dielectric material forming a bulk of the monolithic body at regions from which the magnetic and conductive materials are excluded.
Example 16. The magnetic field sensor according to Example 15, wherein spacings between adjacent turns of the windings are occupied by 3D printed non-electrically-conductive material being one of the non-magnetic dielectric material and the 3D-printed magnetic material; and optionally wherein the conductive material of the windings is fully embedded within the monolithic body so as to be isolated from environmental conditions by which it might be degraded.
Example 17. The magnetic field sensor according to Example 15 or 16, wherein at least one of the following:
   - the conductive material includes Silver;
   - the conductive material includes Copper;
   - the conductive material includes Palladium;
   - the conductive material includes Silver-Palladium alloy;
   - the magnetic material includes Ceramic-Ferrite material;
   - the magnetic material includes Metal Material;
   - the magnetic material is a soft magnetic material having relative permeability µᵣ in the order of 100 or more; and
   - the non-magnetic dielectric material includes ceramic or glass-ceramic material.
Example 18. The magnetic field sensor according to any one of Examples 1 to 17, wherein electric contact terminals of electric channels of the coils are arranged with predetermined arrangement at a surface of the monolithic body, and wherein the magnetic field sensor further comprises a signal connector comprising: a printed circuit board (PCB) with a complementary arrangement of contact pads matching the arrangement of the electric contact terminals of the coils at the surface of the monolithic body and a signal cable with signal lines electrically coupled to the arrangement of contact pads of the PCB; thereby facilitating electrically connection of the magnetic field sensor by coupling said PCB to the surface of the monolithic body such that the arrangement of the electric contact terminals is in matching contact with the complementary arrangement of contact pads of the PCB.
Example 19. A method to fabricate a magnetic field sensor, the method includes 3D printing the magnetic field sensor by successive printing of layers along a printing direction to form a monolithic body of the sensor with one or more longitudinally oriented coils having respective first magnetic axes oriented substantially parallel to the printing direction and one or more transversely oriented coils having respective second magnetic axes oriented transversely to the printing direction.
Example 20. The method according to Example 19, wherein 3D printing of each longitudinally oriented coil includes 3D printing of a first conductive channel forming an arrangement of first conductive windings with turns planarly printed in at least some of the printing layers; and
   wherein 3D printing of each transversely oriented coil includes: 3D printing of a magnetic channel including at least a pair of opposite flux collectors having respective facets perpendicular to the second magnetic axis of the transversely oriented coil and at least one section between them serving as a magnetic core of the transversely oriented coil, and 3D printing of second conductive channel forming an arrangement of second conductive windings 3D printed in the monolithic body with turns surrounding the at least one section serving as the magnetic core;
   whereby the magnetic channel is printed with a curved path such that the at least one sections thereof, which serve as the magnetic core of the transversely oriented coil, extends along the printing direction, substantially perpendicular to the second magnetic axis; and the turns of the second conductive windings are 3D printed planarly within at least some of the printed layers to surround said magnetic core.
Example 21. The method according to Example 20, wherein the pair of flux collectors are 3D printed across a plurality of the printed layers such that they have respective flux collection facets perpendicular to the second magnetic axis for efficient collection of magnetic flux propagating from the direction of the second magnetic axis.
Example 22. The method according to any one of Examples 19 to 21, wherein the 3D printing includes:
   (a) providing curable resins including:
      - magnetic material resin including particles of magnetic material suspended in curable polymer binder;
      - conductive material resin including particles of conductive material suspended in curable polymer binder;
      - non-magnetic dielectric material resin including particles of non-magnetic dielectric material suspended in curable polymer binder; and
   (b) 3D printing said curable resins including:
      - 3D printing and curing the magnetic material resin at regions of the magnetic channel;
      - 3D printing and curing the conductive material resin at regions of the first and second conductive channels; and
      - 3D printing and curing said non-magnetic dielectric material regions of the monolithic body not occupied by the magnetic and conductive channels;
         thereby forming a 3D printed structure of the magnetic field sensor; and
   (c) sintering the 3D printed structure of the magnetic field sensor at temperature sufficient for burning or driving off polymers therefrom and thereby achieving ceramic or metal density approaching 100% in the magnetic field sensor.
Example 23. The method according to any one of Examples 20 to 22, wherein the monolithic body is printed with an elongated shape along the printing direction with relatively short distance between the pair of opposite flux collectors, and wherein the curves of the magnetic channel within the monolithic body provide an extended length of the at least one section serving as the magnetic core, facilitating 3D printing of a higher count of turns of the second conductive windings about the extended length.
Example 24. The method according to any one of Examples 19 to 23, wherein the one or more longitudinally oriented coils include three longitudinally oriented coils of similar magnetic properties 3D printed such that they are coaligned and their respective first magnetic axes are parallel to one another; and
   wherein the one or more transversely oriented coils include two transversely oriented coils 3D printed such that their respective two transversely oriented magnetic axes are not parallel to one another and such that the two transversely oriented magnetic axes, together with a longitudinally oriented magnetic axis of at least one longitudinally oriented coil of the one or more longitudinally oriented coils, span 3D coordinates.
Example 25. The method according to Example 24, wherein the at least three longitudinally oriented coils are 3D printed in respective three angular sectors about a longitudinal axis of the monolithic body which is parallel to the printing direction; and the at least two transversely oriented coils are arranged in respective two angular sectors about the longitudinal axis.
Example 26. The method according to Example 25, wherein a least one of the following: (a) the three angular sectors and the two angular sectors are five coaligned sectors 3D printed about the longitudinal axis of the monolithic body in the same level relative to the longitudinal axis (same height with respect to the relative to the longitudinal axis); and (b) the three angular sectors and the two angular sectors are respectively 3D printed in different levels (different heights) with respect to the longitudinal axis of the monolithic body.
Example 27. The method according to Example 26 or 25, wherein the monolithic body is 3D printed with a cylindrical shape about the longitudinal axis and with characteristic width of about 2 millimeters to enable fitting of the magnetic field sensor within a catheter.
Example 28. The method according to Example 27, wherein the monolithic body is 3D printed with an annular shape and with a hole passing therethrough along the longitudinal axis to facilitate passage of additional components (e.g. electric- and/or fluid-lines) of the catheter through the hole of the sensor; and wherein the a pair of opposite flux collectors of each of the transversely oriented coils are 3D printed such that at least one flux collector is at an external facet of the annular shape and at least one flux collector is at a facet of the annular shape facing the hole.
Example 29. The method according to any one of Examples 20 to 28, wherein the 3D printing provides a higher voxel resolution along the printing direction as compared to lateral voxel resolution within printing layers; and
   wherein the at least one section serving as the magnetic core of the transversely oriented coil extends substantially parallel to the printing direction and the turns of the second conductive windings that surround that at least one section are substantially perpendicular to the printing direction, thereby exploiting the higher voxel resolution along the printing direction to yield high density of the turns of the second conductive windings and improved sensitivity of the transversely oriented coil.
Example 30. The method according to any one of Examples 19 to 29, wherein the at least one magnetic channel of the transversely oriented coil curves in a helical-like or meander-like shape.
Example 31. The method according to any one of Examples 20 to 29, wherein the at least one magnetic channel of the transversely oriented coil curves in a coil shape winded about the second conductive windings.
Example 32. The method according to any one of Examples 20 to 31, wherein the facets of the flux collectors are wider than the at least one section serving as the magnetic core.
Example 33. The method according to any one of Examples 20 to 32, wherein the magnetic channel of the transversely oriented coil is configured such that the transversely oriented coil has an open magnetic circuit configuration.
Example 34. The method according to any one of Examples 19 to 33, wherein 3D printing of at least one longitudinally oriented coil of the one or more longitudinally oriented coils, includes 3D printing a magnetic channel within in the monolithic body, with a pair of opposite flux collectors having respective facets perpendicular to the first magnetic axis and at least one magnetic core section between the pair of opposite flux collectors; and wherein one or more of the following:
   - the pair of flux collectors of the longitudinally oriented coil are 3D printed such that they are tapered towards the magnetic core section of the longitudinally oriented coil;
   - the magnetic core section of the longitudinally oriented coil is 3D printed to extend along the longitudinal direction in the form of a rod;
   - the magnetic channel of the longitudinally oriented coil is 3D printed such that it curves in a coiled shape wrapped about the first conductive windings.
   - the magnetic channel of the longitudinally oriented coil is 3D printed such that it curves within the monolithic body to yield a greater length of the core section that is surrounded by the first windings of the longitudinally oriented coil;
   - the at least one longitudinally oriented coil is 3D printed with an open magnetic circuit configuration; and
   - the pair of opposite flux collectors of the longitudinally oriented coil are 3D printed from opposite longitudinal sides of the monolithic body of the sensor.

## Claims

1. A magnetic field sensor (100) comprising a plurality of coils (120);
**characterized in that** the magnetic field sensor (100) comprises a monolithic body (110) with the plurality of coils (120) defined therein by 3D printing of successive printed layers along a printing direction (Z); the plurality of coils (120) comprises:
- one or more longitudinally oriented coils (120L) having respective first magnetic axes (L) oriented substantially parallel to the printing direction (Z); each of the longitudinally oriented coils (120L) comprises a 3D printed first conductive channel (128L) forming an arrangement of first conductive windings (124L) with turns in planes of said printing layers; and
- said plurality of coils (120) further comprise one or more transversely oriented coils (120T) with respective second magnetic axes (T) transversely oriented relative to said printing direction (Z).

2. The magnetic field sensor (100) according to claim 1, wherein each transversely oriented coil (120T) of the transversely oriented coils comprises a magnetic channel (129T) 3D printed in the monolithic body (110) such that the magnetic channel (129T) comprises at least a pair of opposite flux collectors (126T) having respective facets perpendicular to the second magnetic axis (T) thereof and at least one section between them serving as a magnetic core (122T) of the transversely oriented coil (126T); and a 3D printed second conductive channel (128T) forming an arrangement of second conductive windings (124T) 3D printed in said monolithic body (110) to surround the at least one magnetic channel (129T); and
wherein the magnetic channel (129T) is formed with curves (127) within the monolithic body such that the at least one section thereof, which serves as the magnetic core (122T), extends along said printing direction (Z) and said arrangement of second conductive windings (124T) comprises a plurality of turns printed in planes of said printing layers to surround said at least one section serving as the magnetic core (122T).

3. The magnetic field sensor (100) according to claim 2 wherein said monolithic body has an elongated shape along said printing direction (Z) with relatively short distance between said pair of opposite flux collectors (126T), and wherein said curves (127) of the magnetic channel (129T) within the monolithic body (110) facilitate an extended length of said at least one section serving as the magnetic core (122T) to accommodate higher count of turns of said second conductive windings (124T) about said extended length thereby improving sensitivity of said transversely oriented coils (120T) despite said short distance between the pair of flux collectors (126T).

4. The magnetic field sensor (100) according to any one of claims 1 to 3,
wherein said one or more longitudinally oriented coils (120L) comprise three longitudinally oriented coils (120L.1, 120L.2, and 120L.3) of similar magnetic properties 3D printed such that they are coaligned and their respective first magnetic axes (L1, L2, and L3) are parallel to one another, thereby enabling to utilize signals obtained from the three longitudinally oriented coils (120L.1, 120L.2, and 120L.3) to determine two angles of orientation of the magnetic field sensor (100) relative to a first magnetic field source located in-front of the magnetic field sensor (100) along a general direction of the first magnetic axes (L1, L2, and L3); and
wherein said one or more transversely oriented coils (120T) comprise two transversely oriented coils (120T.1, 120T.2) 3D printed such that their respective two transversely oriented magnetic axes (T1 and T2) are not parallel to one another and such that the two transversely oriented magnetic axes (T1 and T2) together with a longitudinally oriented magnetic axis (L) of at least one longitudinally oriented coil of the one or more longitudinally oriented coils (120L) span 3D coordinates, thereby enabling to utilize signals obtained from the two transversely oriented coils (120T.1, 120T.2) and the at least one longitudinally oriented coil (120L) to determine a location and orientation of the sensor (100) relative to one or more second magnetic field sources.

5. The magnetic field sensor (100) according to claim 4 wherein said at least three longitudinally oriented coils (120L.1, 120L.2, and 120L.3) are arranged in respective three angular sectors about a longitudinal axis of the monolithic body being along said printing direction (Z); and said at least two transversely oriented coils (120T.1 and 120T.2) are arranged in respective two angular sectors about said longitudinal axis; and wherein a least one of the following:
(a) said three angular sectors and said two angular sectors are five coaligned sectors arranged about said longitudinal axis of the monolithic body in the same level; and
(b) said three angular sectors and said two angular sectors are respectively arranged in different levels of the monolithic body.

6. The magnetic field sensor (100) according to claim 5 wherein said monolithic body has a cylindrical shape about said longitudinal axis with characteristic width of about 2 millimeters to thereby enable fitting of said magnetic field sensor within a catheter (1000); and
wherein preferably said monolithic body (110) has an annular shape with a hole (115) passing therethrough along said longitudinal axis to facilitate passage of additional components of the catheter (1000) through said hole (115) of the sensor (100).

7. The magnetic field sensor (100) according to any one of claims 2 to 6 wherein the 3D printing provides a higher voxel resolution along said the printing direction (Z) as compared to lateral voxel resolution within printing layers; and
wherein said at least one section serving as said magnetic core (122T) extends substantially parallel to said printing direction (Z) and said turns of the second conductive windings (124T) that surround said at least one section are substantially perpendicular to said printing direction (Z), thereby exploiting the higher voxel resolution along the printing direction to yield high density of said turns of the second conductive windings (124T) and thereby improved sensitivity of said transversely oriented coil (120T).

8. The magnetic field sensor (100) according to any one of claims 2 to 7) wherein the at least one magnetic channel **(129T)** curves in at least one of:
- a helical-like shape;
- a meander-like shape; and
- a coiled shape winded about the second conductive windings (124T).

9. The magnetic field sensor (100) according to any one of claims 2 to 8 wherein one or more of the following:
- said facets of the flux collectors (126T) are wider than said at least one section serving as the magnetic core (122T).
- said magnetic channel (129T) is configured such that said transversely oriented coil (120T) has an open magnetic circuit configuration.

10. The magnetic field sensor (100) according to any one of claims 1 to 9 wherein at least one longitudinally oriented coil, of the one or more longitudinally oriented coils (120L), comprises a magnetic channel (129L) 3D printed in said monolithic body (110) with a pair of opposite flux collectors (126L) having respective facets perpendicular to the first magnetic axis (L) and at least one magnetic core section (122L) between the pair of opposite flux collectors (126L); and wherein one or more of the following:
- the pair of flux collectors (126L) of the longitudinally oriented coil (120L) are tapered towards the magnetic core section of the longitudinally oriented coil (120L);
- the magnetic core section (122L) of the longitudinally oriented coil (120L) is directed along said longitudinal direction in the form of a rod;
- the magnetic channel (129L) of the longitudinally oriented coil (120L) curves in a coiled shape wrapped about the first conductive windings (124L).
- the magnetic channel (129L) of the longitudinally oriented coil (120L) curves within the monolithic body (110) such that a greater length of said core section (122L) is surrounded by turns of the first conductive windings (124L) of the longitudinally oriented coil (120L) thereby facilitating higher output voltage and improved sensitivity of the longitudinally oriented coil (120L);
- said at least one longitudinally oriented coil (120L) is configured with an open magnetic circuit configuration; and
- the pair of opposite flux collectors (126T) of the longitudinally oriented coil (120L) are arranged from opposite longitudinal sides of the monolithic body (110) of the sensor (100).

11. The magnetic field sensor (100) according to any one of claims 1 to 10 formed by 3D printing of at least three different 3D-printable materials comprising:
- a magnetic material (M1) forming magnetic channels (129) of the coils (120);
- a conductive material (M2) forming electric channels (128) of the coils (120); and
- non-magnetic dielectric material (M3) forming a bulk of said monolithic body (100) at regions from which said magnetic and conductive materials (M1 and M2) are excluded.

12. The magnetic field sensor (100) according to any of claims 1 to 11 wherein electric contact terminals (123) of electric channels (128) of the coils (12) are arranged with predetermined arrangement at a surface of the monolithic body (110), and wherein the magnetic field sensor further comprises a signal connector (150) comprising: a printed circuit board (PCB) with a complementary arrangement of contact pads (153) matching the arrangement of the electric contact terminals (123) of the coils (120) at said surface of the monolithic body (110) and a signal cable (158) with signal lines electrically coupled to the arrangement of contact pads (153) of the PCB (151); thereby facilitating electrically connection of the magnetic field sensor (100) by coupling said PCB (151) to said surface of the monolithic body (110) such that said arrangement of the electric contact terminals (123) is in matching contact with said complementary arrangement of contact pads (153) of the PCB (151).

13. A method to fabricate a magnetic field sensor (100),
the method is **characterized by** 3D printing (200) of the magnetic field sensor by successive printing of layers along a printing direction (Z) to form a monolithic body (110) of the sensor with one or more longitudinally oriented coils (120L) having respective first magnetic axes (L) oriented substantially parallel to the printing direction (Z) and one or more transversely oriented coils (120T) having respective second magnetic axes (T) oriented transversely to the printing direction (Z).

14. The method according to claim 13, wherein the 3D printing (200) of each longitudinally oriented coil comprises 3D printing of a first conductive channel (128L) forming an arrangement of first conductive windings (124L) with turns planarly printed in at least some of said the printing layers; and
wherein 3D printing (200) of each transversely oriented coil (120T) comprises: 3D printing of a magnetic channel (129T) comprising at least a pair of opposite flux collectors (126T) having respective facets perpendicular to the second magnetic axis (T) of the transversely oriented coil (120T) and at least one section between them serving as a magnetic core (122T) of the transversely oriented coil (120T), and 3D printing of second conductive channel (128T) forming an arrangement of second conductive windings (124T) 3D printed in said monolithic body (110) with turns surrounding the at least one section serving as the magnetic core (122T);
whereby said magnetic channel (129T) is printed with a curved path (127) such that said at least one sections thereof, which serve as the magnetic core (122T) of the transversely oriented coil (120T), extends along said printing direction (Z), substantially perpendicular to said second magnetic axis (T); and said turns of the second conductive windings (124T) are 3D printed planarly within at least some of the printed layers to surround said magnetic core (122T).

15. The method according to claim 14 wherein said pair of flux collectors (126T) are 3D printed across a plurality of the printed layers such that they have respective flux collection facets (126F, 126B) perpendicular to the second magnetic axis (T) for efficient collection of magnetic flux propagating from the direction of said second magnetic axis (T).

16. The method according to any one of claims 13 to 15 wherein said 3D printing comprises:
**(a)** providing curable resins (210) comprising:
- magnetic material resin comprising particles of magnetic material (M1) suspended in curable polymer binder;
- conductive material resin comprising particles of conductive material (M2) suspended in curable polymer binder;
- non-magnetic dielectric material resin comprising particles of non-magnetic dielectric material (M3) suspended in curable polymer binder; and
**(b)** 3D printing (220) said curable resins comprising:
- 3D printing and curing said magnetic material resin at regions of magnetic channels (129) of the coils (120);
- 3D printing and curing said conductive material resin at regions of conductive channels (128) of the coils (120); and
- 3D printing and curing said non-magnetic dielectric material resin regions of said monolithic body (110) not occupied by said magnetic and conductive channels (128, 129);
thereby forming a 3D printed structure of the magnetic field sensor (100); and
(c) sintering (230) said 3D printed structure of the magnetic field sensor (100) at temperature sufficient for burning or driving off polymers therefrom and thereby achieving ceramic or metal density approaching 100% in said magnetic field sensor (100).
